# EUROPÄISCHE PATENTSCHRIFT

(11) **EP 1 673 460 B1**
(45) Veröffentlichungstag und Bekanntmachung des Hinweises auf die Patenterteilung: **12.12.2012**
(21) Anmeldenummer: 04790378.6
(22) Anmeldetag: 13.10.2004
(51) Int. Cl.: C12N 15/62, C07K 14/705, C07K 14/47, A61K 38/17, A61K 31/7088

(54) **REKOMBINATE IMPFSTOFFE UND DEREN VERWENDUNG**
RECOMBINANT VACCINES AND USE THEREOF
VACCINS RECOMBINES ET LEUR UTILISATION

(30) Priorität: 14.10.2003 DE 10347710
(43) Veröffentlichungstag der Anmeldung: 28.06.2006
(73) Patentinhaber: BioNTech AG, 55131 Mainz (DE)
(72) Erfinder: TÜRECI, Özlem, 55116 Mainz (DE); SAHIN, Ugur, 55116 Mainz (DE); KREITER, Sebastian, 55131 Mainz (DE)
(74) Vertreter: Schnappauf, Georg
(86) Internationale Anmeldenummer: PCT/EP2004/011512
(87) Internationale Veröffentlichungsnummer: WO 2005/038030

(56) Entgegenhaltungen:
- WO-A-02/080851
- WO-A-2004/015395
- US-A1- 2002 151 707
- NGUYEN P ET AL: "Antigen-specific targeting of CD8+ T cells with receptor-modified T lymphocytes." GENE THERAPY, Bd. 10, Nr. 7, April 2003 (2003-04), Seiten 594-604, XP002314391 ISSN: 0969-7128
- MARQUES ERNESTO T A JR ET AL: "HIV-1 p55Gag encoded in the lysosome-associated membrane protein-1 as a DNA plasmid vaccine chimera is highly expressed, traffics to the major histocompatibility class II compartment, and elicits enhanced immune responses." JOURNAL OF BIOLOGICAL CHEMISTRY, Bd. 278, Nr. 39, 26. September 2003 (2003-09-26), Seiten 37926-37936, XP002314392 ISSN: 0021-9258
- WETTSTEIN D A ET AL: "EXPRESSION OF A CLASS II MAJOR HISTOCOMPATIBILITY COMPLEX MHC HETERODIMER IN A LIPID-LINKED FORM WITH ENHANCED PEPTIDE-SOLUBLE MHC COMPLEX FORMATION AT LOW PH" JOURNAL OF EXPERIMENTAL MEDICINE, Bd. 174, Nr. 1, 1991, Seiten 219-228, XP002314393 ISSN: 0022-1007 in der Anmeldung erwähnt
- MARGALIT A ET AL: "Chimeric beta2 microglobulin/CD3zeta polypeptides expressed in T cells convert MHC class I peptide ligands into T cell activation receptors: A potential tool for specific targeting of pathogenic CD8+ T cells" INTERNATIONAL IMMUNOLOGY, OXFORD UNIVERSITY PRESS, GB, Bd. 15, Nr. 11, November 2003 (2003-11), Seiten 1379-1387, XP009039662 ISSN: 0953-8178

### SEQUENCE LISTING

<110> Johannes Gutenberg-Universität Mainz, vertreten durch den Präsidenten
<120> Rekombinante Impfstoffe und deren Verwendung
<130> 410-1PCT
<150> DE 103 47 710.1
   <151> 2003-10-14
<160> 45
<170> PatentIn version 3.1
<210> 1
   <211> 78
   <212> DNA
   <213> Homo sapiens
<400> 1
   atgcgggtca cggcgccccg aaccctcatc ctgctgctct cgggagccct ggccctgacc 60
   gagacctggg ccggctcc 78
<210> 2
   <211> 26
   <212> PRT
   <213> Homo sapiens
<400> 2
   Met Arg Val Thr Ala Pro Arg Thr Leu Ile Leu Leu Leu Ser Gly Ala
   1 5 10 15
   Leu Ala Leu Thr Glu Thr Trp Ala Gly Ser
   20 25
<210> 3
   <211> 168
   <212> DNA
   <213> Homo sapiens
<400> 3
   atcgtgggca ttgttgctgg cctggctgtc ctagcagttg tggtcatcgg agctgtggtc 60
   gctactgtga tgtgtaggag gaagagctca ggtggaaaag gagggagcta ctctcaggct 120
   gcgtccagcg acagtgccca gggctctgat gtgtctctca cagcttga 168
<210> 4
   <211> 55
   <212> PRT
   <213> Homo sapiens
<400> 4
   Ile Val Gly Ile Val Ala Gly Leu Ala Val Leu Ala Val Val Val Ile
   1 5 10 15
   Gly Ala Val Val Ala Thr Val Met Cys Arg Arg Lys Ser Ser Gly Gly
   20 25 30
   Lys Gly Gly Ser Tyr Ser Gln Ala Ala Ser Ser Asp Ser Ala Gln Gly
   35 40 45
   Ser Asp Val Ser Leu Thr Ala
   50 55
<210> 5
   <211> 129
   <212> DNA
   <213> Homo sapiens
<400> 5
   cagagcaaga tgctgagtgg agtcgggggc tttgtgctgg gcctgctctt ccttggggcc 60
   gggctgttca tctacttcag gaatcagaaa ggacactctg gacttcagcc aagaggattc 120
   ctgagctga 129
<210> 6
   <211> 42
   <212> PRT
   <213> Homo sapiens
<400> 6
   Gln Ser Lys Met Leu Ser Gly Val Gly Gly Phe Val Leu Gly Leu Leu
   1 5 10 15
   Phe Leu Gly Ala Gly Leu Phe Ile Tyr Phe Arg Asn Gln Lys Gly His
   20 25 30
   Ser Gly Leu Gln Pro Arg Gly Phe Leu Ser
   35 40
<210> 7
   <211> 24
   <212> DNA
   <213> künstliche Sequenz
<400> 7
   ctgcaggtcg actctagagg atcc 24
<210> 8
   <211> 8
   <212> PRT
   <213> künstliche Sequenz
<400> 8
   Leu Gln Val Asp Ser Arg Gly Ser
   1 5
<210> 9
   <211> 1683
   <212> DNA
   <213> Cytomegalovirus
<400> 9
   atggagtcgc gcggtcgccg ttgtcccgaa atgatatccg tactgggtcc catttcgggg 60
   cacgtgctga aagccgtgtt tagtcgcggc gatacgccgg tgctgccgca cgagacgcga   120
   ctcctgcaga cgggtatcca cgtacgcgtg agccagccct cgctgatctt ggtatcgcag   180
   tacacgcccg actcgacgcc atgccaccgc ggcgacaatc agctgcaggt gcagcacacg   240
   tactttacgg gcagcgaggt ggagaacgtg tcggtcaacg tgcacaaccc cacgggccga   300
   agcatctgcc ccagccagga gcccatgtcg atctatgtgt acgcgctgcc gctcaagatg   360
   ctgaacatcc ccagcatcaa cgtgcaccac tacccgtcgg cggccgagcg caaacaccga   420
   cacctgcccg tagctgacgc tgtgattcac gcgtcgggca agcagatgtg gcaggcgcgt   480
   ctcacggtct cgggactggc ctggacgcgt cagcagaacc agtggaaaga gcccgacgtc   540
   tactacacgt cagcgttcgt gtttcccacc aaggacgtgg cactgcggca cgtggtgtgc   600
   gcgcacgagc tggtttgctc catggagaac acgcgcgcaa ccaagatgca ggtgataggt   660
   gaccagtacg tcaaggtgta cctggagtcc ttctgcgagg acgtgccctc cggcaagctc   720
   tttatgcacg tcacgctggg ctctgacgtg gaagaggacc tgacgatgac ccgcaacccg   780
   caacccttca tgcgccccca cgagcgcaac ggctttacgg tgttgtgtcc caaaaatatg   840
   ataatcaaac cgggcaagat ctcgcacatc atgctggatg tggcttttac ctcacacgag   900
   cattttgggc tgctgtgtcc caagagcatc ccgggcctga gcatctcagg taacctgttg   960
   atgaacgggc agcagatctt cctggaggta caagccatac gcgagaccgt ggaactgcgt   1020
   cagtacgatc ccgtggctgc gctcttcttt ttcgatatcg acttgctgct gcagcgcggg   1080
   cctcagtaca gcgagcaccc caccttcacc agccagtatc gcatccaggg caagcttgag   1140
   taccgacaca cctgggaccg gcacgacgag ggtgccgccc agggcgacga cgacgtctgg   1200
   accagcggat cggactccga cgaagaactc gtaaccaccg agcgcaagac gccccgcgtc   1260
   accggcggcg gcgccatggc gggcgcctcc acttccgcgg gccgcaaacg caaatcagca   1320
   tcctcggcga cggcgtgcac gtcgggcgtt atgacacgcg gccgccttaa ggccgagtcc   1380
   accgtcgcgc ccgaagagga caccgacgag gattccgaca acgaaatcca caatccggcc   1440
   gtgttcacct ggccgccctg gcaggccggc atcctggccc gcaacctggt gcccatggtg   1500
   gctacggttc agggtcagaa tctgaagtac caggaattct tctgggacgc caacgacatc   1560
   taccgcatct tcgccgaatt ggaaggcgta tggcagcccg ctgcgcaacc caaacgtcgc   1620
   cgccaccggc aagacgcctt gcccgggcca tgcatcgcct cgacgcccaa aaagcaccga   1680
   ggt                                                                 1683
<210> 10
   <211> 561
   <212> PRT
   <213> Cytomegalovirus
<400> 10
   Met Glu Ser Arg Gly Arg Arg Cys Pro Glu Met Ile Ser Val Leu Gly
   1               5                   10                  15
   Pro Ile Ser Gly His Val Leu Lys Ala Val Phe Ser Arg Gly Asp Thr
               20                  25                  30
   Pro Val Leu Pro His Glu Thr Arg Leu Leu Gln Thr Gly Ile His Val
           35                  40                  45
   Arg Val Ser Gln Pro Ser Leu Ile Leu Val Ser Gln Tyr Thr Pro Asp
       50                  55                  60
   Ser Thr Pro Cys His Arg Gly Asp Asn Gln Leu Gln Val Gln His Thr
   65                  70                  75                  80
   Tyr Phe Thr Gly Ser Glu Val Glu Asn Val Ser Val Asn Val His Asn
                   85                  90                  95
   Pro Thr Gly Arg Ser Ile Cys Pro Ser Gln Glu Pro Met Ser Ile Tyr
               100                 105                 110
   Val Tyr Ala Leu Pro Leu Lys Met Leu Asn Ile Pro Ser Ile Asn Val
           115                 120                 125
   His His Tyr Pro Ser Ala Ala Glu Arg Lys His Arg His Leu Pro Val
       130                 135                 140
   Ala Asp Ala Val Ile His Ala Ser Gly Lys Gln Met Trp Gln Ala Arg
   145                 150                 155                 160
   Leu Thr Val Ser Gly Leu Ala Trp Thr Arg Gln Gln Asn Gln Trp Lys
                   165                 170                 175
   Glu Pro Asp Val Tyr Tyr Thr Ser Ala Phe Val Phe Pro Thr Lys Asp
               180                 185                 190
   Val Ala Leu Arg His Val Val Cys Ala His Glu Leu Val Cys Ser Met
           195                 200                 205
   Glu Asn Thr Arg Ala Thr Lys Met Gln Val Ile Gly Asp Gln Tyr Val
       210                 215                 220
   Lys Val Tyr Leu Glu Ser Phe Cys Glu Asp Val Pro Ser Gly Lys Leu
   225                 230                 235                 240
   Phe Met His Val Thr Leu Gly Ser Asp Val Glu Glu Asp Leu Thr Met
                   245                 250                 255
   Thr Arg Asn Pro Gln Pro Phe Met Arg Pro His Glu Arg Asn Gly Phe
               260                 265                 270
   Thr Val Leu Cys Pro Lys Asn Met Ile Ile Lys Pro Gly Lys Ile Ser
           275                 280                 285
   His Ile Met Leu Asp Val Ala Phe Thr Ser His Glu His Phe Gly Leu
   290 295 300
   Leu Cys Pro Lys Ser Ile Pro Gly Leu Ser Ile Ser Gly Asn Leu Leu
   305 310 315 320
   Met Asn Gly Gln Gln Ile Phe Leu Glu Val Gln Ala Ile Arg Glu Thr
   325 330 335
   Val Glu Leu Arg Gln Tyr Asp Pro Val Ala Ala Leu Phe Phe Phe Asp
   340 345 350
   Ile Asp Leu Leu Leu Gln Arg Gly Pro Gln Tyr Ser Glu His Pro Thr
   355 360 365
   Phe Thr Ser Gln Tyr Arg Ile Gln Gly Lys Leu Glu Tyr Arg His Thr
   370 375 380
   Trp Asp Arg His Asp Glu Gly Ala Ala Gln Gly Asp Asp Asp Val Trp
   385 390 395 400
   Thr Ser Gly Ser Asp Ser Asp Glu Glu Leu Val Thr Thr Glu Arg Lys
   405 410 415
   Thr Pro Arg Val Thr Gly Gly Gly Ala Met Ala Gly Ala Ser Thr Ser
   420 425 430
   Ala Gly Arg Lys Arg Lys Ser Ala Ser Ser Ala Thr Ala Cys Thr Ser
   435 440 445
   Gly Val Met Thr Arg Gly Arg Leu Lys Ala Glu Ser Thr Val Ala Pro
   450 455 460
   Glu Glu Asp Thr Asp Glu Asp Ser Asp Asn Glu Ile His Asn Pro Ala
   465 470 475 480
   Val Phe Thr Trp Pro Pro Trp Gln Ala Gly Ile Leu Ala Arg Asn Leu
   485 490 495
   Val Pro Met Val Ala Thr Val Gln Gly Gln Asn Leu Lys Tyr Gln Glu
   500 505 510
   Phe Phe Trp Asp Ala Asn Asp Ile Tyr Arg Ile Phe Ala Glu Leu Glu
   515 520 525
   Gly Val Trp Gln Pro Ala Ala Gln Pro Lys Arg Arg Arg His Arg Gln
   530 535 540
   Asp Ala Leu Pro Gly Pro Cys Ile Ala Ser Thr Pro Lys Lys His Arg
   545 550 555 560
   Gly
<210> 11
   <211> 1962
   <212> DNA
   <213> künstliche Sequenz
<400> 11
   atgcgggtca cggcgccccg aaccctcatc ctgctgctct cgggagccct ggccctgacc      60
   gagacctggg ccggctccct gcaggtcgac tctagaggat ccaccatgga gtcgcgcggt     120
   cgccgttgtc ccgaaatgat atccgtactg ggtcccattt cggggcacgt gctgaaagcc     180
   gtgtttagtc gcggcgatac gccggtgctg ccgcacgaga cgcgactcct gcagacgggt     240
   atccacgtac gcgtgagcca gccctcgctg atcttggtat cgcagtacac gcccgactcg     300
   acgccatgcc accgcggcga caatcagctg caggtgcagc acacgtactt tacgggcagc     360
   gaggtggaga acgtgtcggt caacgtgcac aaccccacgg gccgaagcat ctgccccagc     420
   caggagccca tgtcgatcta tgtgtacgcg ctgccgctca agatgctgaa catccccagc     480
   atcaacgtgc accactaccc gtcggcggcc gagcgcaaac accgacacct gcccgtagct     540
   gacgctgtga ttcacgcgtc gggcaagcag atgtggcagg cgcgtctcac ggtctcggga     600
   ctggcctgga cgcgtcagca gaaccagtgg aaagagcccg acgtctacta cacgtcagcg     660
   ttcgtgtttc ccaccaagga cgtggcactg cggcacgtgg tgtgcgcgca cgagctggtt     720
   tgctccatgg agaacacgcg cgcaaccaag atgcaggtga taggtgacca gtacgtcaag     780
   gtgtacctgg agtccttctg cgaggacgtg ccctccggca agctctttat gcacgtcacg     840
   ctgggctctg acgtggaaga ggacctgacg atgacccgca acccgcaacc cttcatgcgc     900
   ccccacgagc gcaacggctt tacggtgttg tgtcccaaaa atatgataat caaaccgggc     960
   aagatctcgc acatcatgct ggatgtggct tttacctcac acgagcattt tgggctgctg    1020
   tgtcccaaga gcatcccggg cctgagcatc tcaggtaacc tgttgatgaa cgggcagcag    1080
   atcttcctgg aggtacaagc catacgcgag accgtggaac tgcgtcagta cgatcccgtg    1140
   gctgcgctct tctttttcga tatcgacttg ctgctgcagc gcgggcctca gtacagcgag    1200
   caccccacct tcaccagcca gtatcgcatc cagggcaagc ttgagtaccg acacacctgg    1260
   gaccggcacg acgagggtgc cgcccagggc gacgacgacg tctggaccag cggatcggac    1320
   tccgacgaag aactcgtaac caccgagcgc aagacgcccc gcgtcaccgg cggcggcgcc    1380
   atggcgggcg cctccacttc cgcgggccgc aaacgcaaat cagcatcctc ggcgacggcg    1440
   tgcacgtcgg gcgttatgac acgcggccgc cttaaggccg agtccaccgt cgcgcccgaa    1500
   gaggacaccg acgaggattc cgacaacgaa atccacaatc cggccgtgtt cacctggccg    1560
   ccctggcagg ccggcatcct ggcccgcaac ctggtgccca tggtggctac ggttcagggt    1620
   cagaatctga agtaccagga attcttctgg gacgccaacg acatctaccg catcttcgcc    1680
   gaattggaag gcgtatggca gcccgctgcg caacccaaac gtcgccgcca ccggcaagac    1740
   gccttgcccg ggccatgcat cgcctcgacg cccaaaaagc accgaggtgg atccatcgtg 1800
   ggcattgttg ctggcctggc tgtcctagca gttgtggtca tcggagctgt ggtcgctact 1860
   gtgatgtgta ggaggaagag ctcaggtgga aaaggaggga gctactctca ggctgcgtcc 1920
   agcgacagtg cccagggctc tgatgtgtct ctcacagctt ga 1962
<210> 12
   <211> 653
   <212> PRT
   <213> künstliche Sequenz
<400> 12
   Met Arg Val Thr Ala Pro Arg Thr Leu Ile Leu Leu Leu Ser Gly Ala
   1               5                   10                  15
   Leu Ala Leu Thr Glu Thr Trp Ala Gly Ser Leu Gln Val Asp Ser Arg
               20                  25                  30
   Gly Ser Thr Met Glu Ser Arg Gly Arg Arg Cys Pro Glu Met Ile Ser
           35                  40                  45
   Val Leu Gly Pro Ile Ser Gly His Val Leu Lys Ala Val Phe Ser Arg
       50                  55                  60
   Gly Asp Thr Pro Val Leu Pro His Glu Thr Arg Leu Leu Gln Thr Gly
   65                  70                  75                  80
   Ile His Val Arg Val Ser Gln Pro Ser Leu Ile Leu Val Ser Gln Tyr
                   85                  90                  95
   Thr Pro Asp Ser Thr Pro Cys His Arg Gly Asp Asn Gln Leu Gln Val
               100                 105                 110
   Gln His Thr Tyr Phe Thr Gly Ser Glu Val Glu Asn Val Ser Val Asn
           115                 120                 125
   Val His Asn Pro Thr Gly Arg Ser Ile Cys Pro Ser Gln Glu Pro Met
       130                 135                 140
   Ser Ile Tyr Val Tyr Ala Leu Pro Leu Lys Met Leu Asn Ile Pro Ser
   145                 150                 155                 160
   Ile Asn Val His His Tyr Pro Ser Ala Ala Glu Arg Lys His Arg His
                   165                 170                 175
   Leu Pro Val Ala Asp Ala Val Ile His Ala Ser Gly Lys Gln Met Trp
               180                 185                 190
   Gln Ala Arg Leu Thr Val Ser Gly Leu Ala Trp Thr Arg Gln Gln Asn
           195                 200                 205
   Gln Trp Lys Glu Pro Asp Val Tyr Tyr Thr Ser Ala Phe Val Phe Pro
   210 215 220
   Thr Lys Asp Val Ala Leu Arg His Val Val Cys Ala His Glu Leu Val
   225 230 235 240
   Cys Ser Met Glu Asn Thr Arg Ala Thr Lys Met Gln Val Ile Gly Asp
   245 250 255
   Gln Tyr Val Lys Val Tyr Leu Glu Ser Phe Cys Glu Asp Val Pro Ser
   260 265 270
   Gly Lys Leu Phe Met His Val Thr Leu Gly Ser Asp Val Glu Glu Asp
   275 280 285
   Leu Thr Met Thr Arg Asn Pro Gln Pro Phe Met Arg Pro His Glu Arg
   290 295 300
   Asn Gly Phe Thr Val Leu Cys Pro Lys Asn Met Ile Ile Lys Pro Gly
   305 310 315 320
   Lys Ile Ser His Ile Met Leu Asp Val Ala Phe Thr Ser His Glu His
   325 330 335
   Phe Gly Leu Leu Cys Pro Lys Ser Ile Pro Gly Leu Ser Ile Ser Gly
   340 345 350
   Asn Leu Leu Met Asn Gly Gln Gln Ile Phe Leu Glu Val Gln Ala Ile
   355 360 365
   Arg Glu Thr Val Glu Leu Arg Gln Tyr Asp Pro Val Ala Ala Leu Phe
   370 375 380
   Phe Phe Asp Ile Asp Leu Leu Leu Gln Arg Gly Pro Gln Tyr Ser Glu
   385 390 395 400
   His Pro Thr Phe Thr Ser Gln Tyr Arg Ile Gln Gly Lys Leu Glu Tyr
   405 410 415
   Arg His Thr Trp Asp Arg His Asp Glu Gly Ala Ala Gln Gly Asp Asp
   420 425 430
   Asp Val Trp Thr Ser Gly Ser Asp Ser Asp Glu Glu Leu Val Thr Thr
   435 440 445
   Glu Arg Lys Thr Pro Arg Val Thr Gly Gly Gly Ala Met Ala Gly Ala
   450 455 460
   Ser Thr Ser Ala Gly Arg Lys Arg Lys Ser Ala Ser Ser Ala Thr Ala
   465 470 475 480
   Cys Thr Ser Gly Val Met Thr Arg Gly Arg Leu Lys Ala Glu Ser Thr
   485 490 495
   Val Ala Pro Glu Glu Asp Thr Asp Glu Asp Ser Asp Asn Glu Ile His
   500 505 510
   Asn Pro Ala Val Phe Thr Trp Pro Pro Trp Gln Ala Gly Ile Leu Ala
   515 520 525
   Arg Asn Leu Val Pro Met Val Ala Thr Val Gln Gly Gln Asn Leu Lys
   530 535 540
   Tyr Gln Glu Phe Phe Trp Asp Ala Asn Asp Ile Tyr Arg Ile Phe Ala
   545 550 555 560
   Glu Leu Glu Gly Val Trp Gln Pro Ala Ala Gln Pro Lys Arg Arg Arg
   565 570 575
   His Arg Gln Asp Ala Leu Pro Gly Pro Cys Ile Ala Ser Thr Pro Lys
   580 585 590
   Lys His Arg Gly Gly Ser Ile Val Gly Ile Val Ala Gly Leu Ala Val
   595 600 605
   Leu Ala Val Val Val Ile Gly Ala Val Val Ala Thr Val Met Cys Arg
   610 615 620
   Arg Lys Ser Ser Gly Gly Lys Gly Gly Ser Tyr Ser Gln Ala Ala Ser
   625 630 635 640
   Ser Asp Ser Ala Gln Gly Ser Asp Val Ser Leu Thr Ala
   645 650
<210> 13
   <211> 1923
   <212> DNA
   <213> künstliche Sequenz
<400> 13
   atgcgggtca cggcgccccg aaccctcatc ctgctgctct cgggagccct ggccctgacc 60
   gagacctggg ccggctccct gcaggtcgac tctagaggat ccaccatgga gtcgcgcggt 120
   cgccgttgtc ccgaaatgat atccgtactg ggtcccattt cggggcacgt gctgaaagcc 180
   gtgtttagtc gcggcgatac gccggtgctg ccgcacgaga cgcgactcct gcagacgggt 240
   atccacgtac gcgtgagcca gccctcgctg atcttggtat cgcagtacac gcccgactcg 300
   acgccatgcc accgcggcga caatcagctg caggtgcagc acacgtactt tacgggcagc 360
   gaggtggaga acgtgtcggt caacgtgcac aaccccacgg gccgaagcat ctgccccagc 420
   caggagccca tgtcgatcta tgtgtacgcg ctgccgctca agatgctgaa catccccagc 480
   atcaacgtgc accactaccc gtcggcggcc gagcgcaaac accgacacct gcccgtagct 540
   gacgctgtga ttcacgcgtc gggcaagcag atgtggcagg cgcgtctcac ggtctcggga 600
   ctggcctgga cgcgtcagca gaaccagtgg aaagagcccg acgtctacta cacgtcagcg 660
   ttcgtgtttc ccaccaagga cgtggcactg cggcacgtgg tgtgcgcgca cgagctggtt 720
   tgctccatgg agaacacgcg cgcaaccaag atgcaggtga taggtgacca gtacgtcaag 780
   gtgtacctgg agtccttctg cgaggacgtg ccctccggca agctctttat gcacgtcacg 840
   ctgggctctg acgtggaaga ggacctgacg atgacccgca acccgcaacc cttcatgcgc 900
   ccccacgagc gcaacggctt tacggtgttg tgtcccaaaa atatgataat caaaccgggc 960
   aagatctcgc acatcatgct ggatgtggct tttacctcac acgagcattt tgggctgctg 1020
   tgtcccaaga gcatcccggg cctgagcatc tcaggtaacc tgttgatgaa cgggcagcag 1080
   atcttcctgg aggtacaagc catacgcgag accgtggaac tgcgtcagta cgatcccgtg 1140
   gctgcgctct tctttttcga tatcgacttg ctgctgcagc gcgggcctca gtacagcgag 1200
   caccccacct tcaccagcca gtatcgcatc cagggcaagc ttgagtaccg acacacctgg 1260
   gaccggcacg acgagggtgc cgcccagggc gacgacgacg tctggaccag cggatcggac 1320
   tccgacgaag aactcgtaac caccgagcgc aagacgcccc gcgtcaccgg cggcggcgcc 1380
   atggcgggcg cctccacttc cgcgggccgc aaacgcaaat cagcatcctc ggcgacggcg 1440
   tgcacgtcgg gcgttatgac acgcggccgc cttaaggccg agtccaccgt cgcgcccgaa 1500
   gaggacaccg acgaggattc cgacaacgaa atccacaatc cggccgtgtt cacctggccg 1560
   ccctggcagg ccggcatcct ggcccgcaac ctggtgccca tggtggctac ggttcagggt 1620
   cagaatctga agtaccagga attcttctgg gacgccaacg acatctaccg catcttcgcc 1680
   gaattggaag gcgtatggca gcccgctgcg caacccaaac gtcgccgcca ccggcaagac 1740
   gccttgcccg ggccatgcat cgcctcgacg cccaaaaagc accgaggtgg atcccagagc 1800
   aagatgctga gtggagtcgg gggctttgtg ctgggcctgc tcttccttgg ggccgggctg 1860
   ttcatctact tcaggaatca gaaaggacac tctggacttc agccaagagg attcctgagc 1920
   tga 1923
<210> 14
   <211> 640
   <212> PRT
   <213> künstliche Sequenz
<400> 14
   Met Arg Val Thr Ala Pro Arg Thr Leu Ile Leu Leu Leu Ser Gly Ala
   1 5 10 15
   Leu Ala Leu Thr Glu Thr Trp Ala Gly Ser Leu Gln Val Asp Ser Arg
   20 25 30
   Gly Ser Thr Met Glu Ser Arg Gly Arg Arg Cys Pro Glu Met Ile Ser
   35 40 45
   Val Leu Gly Pro Ile Ser Gly His Val Leu Lys Ala Val Phe Ser Arg
       50                  55                  60
   Gly Asp Thr Pro Val Leu Pro His Glu Thr Arg Leu Leu Gln Thr Gly
   65                  70                  75                  80
   Ile His Val Arg Val Ser Gln Pro Ser Leu Ile Leu Val Ser Gln Tyr
                   85                  90                  95
   Thr Pro Asp Ser Thr Pro Cys His Arg Gly Asp Asn Gln Leu Gln Val
               100                 105                 110
   Gln His Thr Tyr Phe Thr Gly Ser Glu Val Glu Asn Val Ser Val Asn
           115                 120                 125
   Val His Asn Pro Thr Gly Arg Ser Ile Cys Pro Ser Gln Glu Pro Met
       130                 135                 140
   Ser Ile Tyr Val Tyr Ala Leu Pro Leu Lys Met Leu Asn Ile Pro Ser
   145                 150                 155                 160
   Ile Asn Val His His Tyr Pro Ser Ala Ala Glu Arg Lys His Arg His
                   165                 170                 175
   Leu Pro Val Ala Asp Ala Val Ile His Ala Ser Gly Lys Gln Met Trp
               180                 185                 190
   Gln Ala Arg Leu Thr Val Ser Gly Leu Ala Trp Thr Arg Gln Gln Asn
           195                 200                 205
   Gln Trp Lys Glu Pro Asp Val Tyr Tyr Thr Ser Ala Phe Val Phe Pro
       210                 215                 220
   Thr Lys Asp Val Ala Leu Arg His Val Val Cys Ala His Glu Leu Val
   225                 230                 235                 240
   Cys Ser Met Glu Asn Thr Arg Ala Thr Lys Met Gln Val Ile Gly Asp
                   245                 250                 255
   Gln Tyr Val Lys Val Tyr Leu Glu Ser Phe Cys Glu Asp Val Pro Ser
               260                 265                 270
   Gly Lys Leu Phe Met His Val Thr Leu Gly Ser Asp Val Glu Glu Asp
           275                 280                 285
   Leu Thr Met Thr Arg Asn Pro Gln Pro Phe Met Arg Pro His Glu Arg
       290                 295                 300
   Asn Gly Phe Thr Val Leu Cys Pro Lys Asn Met Ile Ile Lys Pro Gly
   305                 310                 315                 320
   Lys Ile Ser His Ile Met Leu Asp Val Ala Phe Thr Ser His Glu His
   325 330 335
   Phe Gly Leu Leu Cys Pro Lys Ser Ile Pro Gly Leu Ser Ile Ser Gly
   340 345 350
   Asn Leu Leu Met Asn Gly Gln Gln Ile Phe Leu Glu Val Gln Ala Ile
   355 360 365
   Arg Glu Thr Val Glu Leu Arg Gln Tyr Asp Pro Val Ala Ala Leu Phe
   370 375 380
   Phe Phe Asp Ile Asp Leu Leu Leu Gln Arg Gly Pro Gln Tyr Ser Glu
   385 390 395 400
   His Pro Thr Phe Thr Ser Gln Tyr Arg Ile Gln Gly Lys Leu Glu Tyr
   405 410 415
   Arg His Thr Trp Asp Arg His Asp Glu Gly Ala Ala Gln Gly Asp Asp
   420 425 430
   Asp Val Trp Thr Ser Gly Ser Asp Ser Asp Glu Glu Leu Val Thr Thr
   435 440 445
   Glu Arg Lys Thr Pro Arg Val Thr Gly Gly Gly Ala Met Ala Gly Ala
   450 455 460
   Ser Thr Ser Ala Gly Arg Lys Arg Lys Ser Ala Ser Ser Ala Thr Ala
   465 470 475 480
   Cys Thr Ser Gly Val Met Thr Arg Gly Arg Leu Lys Ala Glu Ser Thr
   485 490 495
   Val Ala Pro Glu Glu Asp Thr Asp Glu Asp Ser Asp Asn Glu Ile His
   500 505 510
   Asn Pro Ala Val Phe Thr Trp Pro Pro Trp Gln Ala Gly Ile Leu Ala
   515 520 525
   Arg Asn Leu Val Pro Met Val Ala Thr Val Gln Gly Gln Asn Leu Lys
   530 535 540
   Tyr Gln Glu Phe Phe Trp Asp Ala Asn Asp Ile Tyr Arg Ile Phe Ala
   545 550 555 560
   Glu Leu Glu Gly Val Trp Gln Pro Ala Ala Gln Pro Lys Arg Arg Arg
   565 570 575
   His Arg Gln Asp Ala Leu Pro Gly Pro Cys Ile Ala Ser Thr Pro Lys
   580 585 590
   Lys His Arg Gly Gly Ser Gln Ser Lys Met Leu Ser Gly Val Gly Gly
   595 600 605
   Phe Val Leu Gly Leu Leu Phe Leu Gly Ala Gly Leu Phe Ile Tyr Phe
   610 615 620
   Arg Asn Gln Lys Gly His Ser Gly Leu Gln Pro Arg Gly Phe Leu Ser
   625 630 635 640
<210> 15
   <211> 66
   <212> PRT
   <213> Homo sapiens
<400> 15
   Pro Ser Ser Gln Pro Thr Ile Pro Ile Val Gly Ile Ile Ala Gly Leu
   1 5 10 15
   Val Leu Phe Gly Ala Val Ile Thr Gly Ala Val Val Ala Ala Val Met
   20 25 30
   Trp Arg Arg Lys Ser Ser Asp Arg Lys Gly Gly Ser Tyr Ser Gln Ala
   35 40 45
   Ala Ser Ser Asp Ser Ala Gln Gly Ser Asp Val Ser Leu Thr Ala Cys
   50 55 60
   Lys Val
   65
<210> 16
   <211> 24
   <212> PRT
   <213> Homo sapiens
<400> 16
   Gly Ser Tyr Ser Gln Ala Ala Ser Ser Asp Ser Ala Gln Gly Ser Asp
   1 5 10 15
   Val Ser Leu Thr Ala Cys Lys Val
   20
<210> 17
   <211> 63
   <212> PRT
   <213> Homo sapiens
<400> 17
   Pro Ser Ser Gln Ser Thr Val Pro Ile Val Gly Ile Val Ala Gly Leu
   1               5                   10                  15
   Ala Val Leu Ala Val Val Val Ile Gly Ala Val Val Ala Ala Val Met
               20                  25                  30
   Cys Arg Arg Lys Ser Ser Gly Gly Lys Gly Gly Ser Tyr Ser Gln Ala
           35                  40                  45
   Ala Cys Ser Asp Ser Ala Gln Gly Ser Asp Val Ser Leu Thr Ala
       50                  55                  60
<210> 18
   <211> 21
   <212> PRT
   <213> Homo sapiens
<400> 18
   Gly Ser Tyr Ser Gln Ala Ala Cys Ser Asp Ser Ala Gln Gly Ser Asp
   1               5                   10                  15
   Val Ser Leu Thr Ala
               20
<210> 19
   <211> 67
   <212> PRT
   <213> Homo sapiens
<400> 19
   Pro Ser Ser Gln Pro Thr Ile Pro Ile Val Gly Ile Val Ala Gly Leu
   1               5                   10                  15
   Ala Val Leu Ala Val Leu Ala Val Leu Gly Ala Met Val Ala Val Val
               20                  25                  30
   Met Cys Arg Arg Lys Ser Ser Gly Gly Lys Gly Gly Ser Cys Ser Gln
           35                  40                  45
   Ala Ala Ser Ser Asn Ser Ala Gln Gly Ser Asp Glu Ser Leu Ile Ala
       50                  55                  60
   Cys Lys Ala
   65
<210> 20
   <211> 14
   <212> PRT
   <213> Homo sapiens
<400> 20
   Ser Ala Gln Gly Ser Asp Glu Ser Leu Ile Ala Cys Lys Ala
   1 5 10
<210> 21
   <211> 62
   <212> PRT
   <213> Homo sapiens
<400> 21
   Pro Ala Ser Gln Pro Thr Ile Pro Ile Val Gly Ile Ile Ala Gly Leu
   1 5 10 15
   Val Leu Leu Gly Ser Val Val Ser Gly Ala Val Val Ala Ala Val Ile
   20 25 30
   Trp Arg Lys Lys Ser Ser Gly Gly Lys Gly Gly Ser Tyr Ser Lys Ala
   35 40 45
   Glu Trp Ser Asp Ser Ala Gln Gly Ser Glu Ser His Ser Leu
   50 55 60
<210> 22
   <211> 20
   <212> PRT
   <213> Homo sapiens
<400> 22
   Gly Ser Tyr Ser Lys Ala Glu Trp Ser Asp Ser Ala Gln Gly Ser Glu
   1 5 10 15
   Ser His Ser Leu
   20
<210> 23
   <211> 66
   <212> PRT
   <213> Homo sapiens
<400> 23
   Gln Ser Pro Gln Pro Thr Ile Pro Ile Val Gly Ile Val Ala Gly Leu
   1 5 10 15
   Val Val Leu Gly Ala Val Val Thr Gly Ala Val Val Ala Ala Val Met
   20 25 30
   Trp Arg Lys Lys Ser Ser Asp Arg Asn Arg Gly Ser Tyr Ser Gln Ala
   35 40 45
   Ala Val Thr Asp Ser Ala Gln Gly Ser Gly Val Ser Leu Thr Ala Asn
   50 55 60
   Lys Val
   65
<210> 24
   <211> 27
   <212> PRT
   <213> Homo sapiens
<400> 24
   Arg Asn Arg Gly Ser Tyr Ser Gln Ala Ala Val Thr Asp Ser Ala Gln
   1 5 10 15
   Gly Ser Gly Val Ser Leu Thr Ala Asn Lys Val
   20 25
<210> 25
   <211> 37
   <212> PRT
   <213> Homo sapiens
<400> 25
   Val Val Cys Ala Leu Gly Leu Thr Val Gly Leu Val Gly Ile Ile Ile
   1 5 10 15
   Gly Thr Ile Phe Ile Ile Lys Gly Leu Arg Lys Ser Asn Ala Ala Glu
   20 25 30
   Arg Arg Gly Pro Leu
   35
<210> 26
   <211> 12
   <212> PRT
   <213> Homo sapiens
<400> 26
   Arg Lys Ser Asn Ala Ala Glu Arg Arg Gly Pro Leu
   1 5 10
<210> 27
   <211> 38
   <212> PRT
   <213> Homo sapiens
<400> 27
   Met Leu Ser Gly Val Gly Gly Phe Val Leu Gly Leu Leu Phe Leu Ala
   1               5                   10                  15
   Gly Leu Phe Ile Tyr Phe Arg Asn Gln Lys Gly His Ser Gly Leu Gln
               20                  25                  30
   Pro Arg Gly Phe Leu Ser
           35
<210> 28
   <211> 12
   <212> PRT
   <213> Homo sapiens
<400> 28
   Gly His Ser Gly Leu Gln Pro Arg Gly Phe Leu Ser
   1               5                   10
<210> 29
   <211> 37
   <212> PRT
   <213> Homo sapiens
<400> 29
   Val Val Cys Ala Leu Gly Leu Ser Val Gly Leu Met Gly Ile Val Val
   1               5                   10                  15
   Gly Thr Val Phe Ile Ile Gln Gly Leu Arg Ser Val Gly Ala Ser Arg
               20                  25                  30
   His Gln Gly Pro Leu
           35
<210> 30
   <211> 10
   <212> PRT
   <213> Homo sapiens
<400> 30
   Val Gly Ala Ser Arg His Gln Gly Pro Leu
   1               5                   10
<210> 31
   <211> 31
   <212> PRT
   <213> Homo sapiens
<400> 31
   Met Leu Ser Gly Ile Gly Gly Phe Val Leu Gly Leu Ile Phe Leu Gly
   1               5                   10                  15
   Leu Gly Leu Ile Ile His His Arg Ser Gln Lys Gly Leu Leu His
               20                  25                  30
<210> 32
   <211> 8
   <212> PRT
   <213> Homo sapiens
<400> 32
   Arg Ser Gln Lys Gly Leu Leu His
   1               5
<210> 33
   <211> 37
   <212> PRT
   <213> Homo sapiens
<400> 33
   Val Leu Cys Ala Leu Gly Leu Val Leu Gly Leu Val Gly Ile Ile Val
   1               5                   10                  15
   Gly Thr Val Leu Ile Ile Lys Ser Leu Arg Ser Gly His Asp Pro Arg
               20                  25                  30
   Ala Gln Gly Thr Leu
           35
<210> 34
   <211> 12
   <212> PRT
   <213> Homo sapiens
<400> 34
   Arg Ser Gly His Asp Pro Arg Ala Gln Gly Thr Leu
   1               5                   10
<210> 35
   <211> 33
   <212> PRT
   <213> Homo sapiens
<400> 35
   Thr Leu Thr Gly Ala Gly Gly Phe Val Leu Gly Leu Ile Ile Cys Gly
   1 5 10 15
   Val Gly Ile Phe Met His Arg Arg Ser Lys Lys Val Gln Arg Gly Ser
   20 25 30
   Ala
<210> 36
   <211> 9
   <212> PRT
   <213> Homo sapiens
<400> 36
   Ser Lys Lys Val Gln Arg Gly Ser Ala
   1 5
<210> 37
   <211> 26
   <212> PRT
   <213> Homo sapiens
<400> 37
   Phe Ile Ile Leu Ala Val Ile Val Pro Leu Leu Leu Leu Ile Gly Leu
   1 5 10 15
   Ala Leu Trp Phe Arg Lys Arg Cys Phe Cys
   20 25
<210> 38
   <211> 6
   <212> PRT
   <213> Homo sapiens
<400> 38
   Arg Lys Arg Cys Phe Cys
   1 5
<210> 39
   <211> 30
   <212> PRT
   <213> Homo sapiens
<400> 39
   Ile Val Leu Ala Ile Ile Val Pro Ser Leu Leu Leu Leu Leu Cys Leu
   1 5 10 15
   Ala Leu Trp Tyr Met Arg Arg Arg Ser Tyr Gln Asn Ile Pro
   20 25 30
<210> 40
   <211> 9
   <212> PRT
   <213> Homo sapiens
<400> 40
   Arg Arg Arg Ser Tyr Gln Asn Ile Pro
   1               5
<210> 41
   <211> 30
   <212> PRT
   <213> Homo sapiens
<400> 41
   Trp Ile Ala Leu Val Val Ile Val Pro Leu Val Ile Leu Ile Val Leu
   1               5                   10                  15
   Val Leu Trp Phe Lys Lys His Cys Ser Tyr Gln Asp Ile Leu
               20                  25                  30
<210> 42
   <211> 10
   <212> PRT
   <213> Homo sapiens
<400> 42
   Lys Lys His Cys Ser Tyr Gln Asp Ile Leu
   1               5                   10
<210> 43
   <211> 1527
   <212> DNA
   <213> Homo sapiens
<400> 43
   atggaacgaa ggcgtttgtg gggttccatt cagagccgat acatcagcat gagtgtgtgg 60
   acaagcccac ggagacttgt ggagctggca gggcagagcc tgctgaagga tgaggccctg 120
   gccattgccg ccctggagtt gctgcccagg gagctcttcc cgccactctt catggcagcc 180
   tttgacggga gacacagcca gaccctgaag gcaatggtgc aggcctggcc cttcacctgc 240
   ctccctctgg gagtgctgat gaagggacaa catcttcacc tggagacctt caaagctgtg 300
   cttgatggac ttgatgtgct ccttgcccag gaggttcgcc ccaggaggtg gaaacttcaa 360
   gtgctggatt tacggaagaa ctctcatcag gacttctgga ctgtatggtc tggaaacagg 420
   gccagtctgt actcatttcc agagccagaa gcagctcagc ccatgacaaa gaagcgaaaa 480
   gtagatggtt tgagcacaga ggcagagcag cccttcattc cagtagaggt gctcgtagac 540
   ctgttcctca aggaaggtgc ctgtgatgaa ttgttctcct acctcattga gaaagtgaag 600
   cgaaagaaaa atgtactacg cctgtgctgt aagaagctga agatttttgc aatgcccatg 660
   caggatatca agatgatcct gaaaatggtg cagctggact ctattgaaga tttggaagtg 720
   acttgtacct ggaagctacc caccttggcg aaattttctc cttacctggg ccagatgatt 780
   aatctgcgta gactcctcct ctcccacatc catgcatctt cctacatttc cccggagaag 840
   gaagagcagt atatcgccca gttcacctct cagttcctca gtctgcagtg cctgcaggct 900
   ctctatgtgg actctttatt tttccttaga ggccgcctgg atcagttgct caggcacgtg 960
   atgaacccct tggaaaccct ctcaataact aactgccggc tttcggaagg ggatgtgatg 1020
   catctgtccc agagtcccag cgtcagtcag ctaagtgtcc tgagtctaag tggggtcatg 1080
   ctgaccgatg taagtcccga gcccctccaa gctctgctgg agagagcctc tgccaccctc 1140
   caggacctgg tctttgatga gtgtgggatc acggatgatc agctccttgc cctcctgcct 1200
   tccctgagcc actgctccca gcttacaacc ttaagcttct acgggaattc catctccata 1260
   tctgccttgc agagtctcct gcagcacctc atcgggctga gcaatctgac ccacgtgctg 1320
   tatcctgtcc ccctggagag ttatgaggac atccatggta ccctccacct ggagaggctt 1380
   gcctatctgc atgccaggct cagggagttg ctgtgtgagt tggggcggcc cagcatggtc 1440
   tggcttagtg ccaacccctg tcctcactgt ggggacagaa ccttctatga cccggagccc 1500
   atcctgtgcc cctgtttcat gcctaac 1527
<210> 44
   <211> 1296
   <212> DNA
   <213> Homo sapiens
<400> 44
   atgggctccg acgtgcggga cctgaacgcg ctgctgcccg ccgtcccctc cctgggtggc 60
   ggcggcggct gtgccctgcc tgtgagcggc gcggcgcagt gggcgccggt gctggacttt    120
   gcgcccccgg gcgcttcggc ttacgggtcg ttgggcggcc ccgcgccgcc accggctccg    180
   ccgccacccc cgccgccgcc gcctcactcc ttcatcaaac aggagccgag ctggggcggc    240
   gcggagccgc acgaggagca gtgcctgagc gccttcactg tccacttttc cggccagttc    300
   actggcacag ccggagcctg tcgctacggg cccttcggtc ctcctccgcc cagccaggcg    360
   tcatccggcc aggccaggat gtttcctaac gcgccctacc tgcccagctg cctcgagagc    420
   cagcccgcta ttcgcaatca gggttacagc acggtcacct tcgacgggac gcccagctac    480
   ggtcacacgc cctcgcacca tgcggcgcag ttccccaacc actcattcaa gcatgaggat    540
   cccatgggcc agcagggctc gctgggtgag cagcagtact cggtgccgcc cccggtctat    600
   ggctgccaca cccccaccga cagctgcacc ggcagccagg ctttgctgct gaggacgccc    660
   tacagcagtg acaatttata ccaaatgaca tcccagcttg aatgcatgac ctggaatcag    720
   atgaacttag gagccacctt aaagggccac agcacagggt acgagagcga taaccacaca    780
   acgcccatcc tctgcggagc ccaatacaga atacacacgc acggtgtctt cagaggcatt    840
   caggatgtgc gacgtgtgcc tggagtagcc ccgactcttg tacggtcggc atctgagacc    900
   agtgagaaac gccccttcat gtgtgcttac ccaggctgca ataagagata ttttaagctg    960
   tcccacttac agatgcacag caggaagcac actggtgaga aaccatacca gtgtgacttc   1020
   aaggactgtg aacgaaggtt ttctcgttca gaccagctca aaagacacca aaggagacat   1080
   acaggtgtga aaccattcca gtgtaaaact tgtcagcgaa agttctcccg gtccgaccac   1140
   ctgaagaccc acaccaggac tcatacaggt aaaacaagtg aaaagccctt cagctgtcgg   1200
   tggccaagtt gtcagaaaaa gtttgcccgg tcagatgaat tagtccgcca tcacaacatg   1260
   catcagagaa acatgaccaa actccagctg gcgctt                             1296
<210> 45
   <211> 1179
   <212> DNA
   <213> Homo sapiens
<400> 45
   atggaggagc cgcagtcaga tcctagcgtc gagccccctc tgagtcagga aacattttca     60
   gacctatgga aactacttcc tgaaaacaac gttctgtccc ccttgccgtc ccaagcaatg    120
   gatgatttga tgctgtcccc ggacgatatt gaacaatggt tcactgaaga cccaggtcca    180
   gatgaagctc ccagaatgcc agaggctgct ccccgcgtgg cccctgcacc agcagctcct    240
   acaccggcgg cccctgcacc agccccctcc tggcccctgt catcttctgt cccttcccag    300
   aaaacctacc agggcagcta cggtttccgt ctgggcttct tgcattctgg gacagccaag    360
   tctgtgactt gcacgtactc ccctgccctc aacaagatgt tttgccaact ggccaagacc    420
   tgccctgtgc agctgtgggt tgattccaca cccccgcccg gcacccgcgt ccgcgccatg    480
   gccatctaca agcagtcaca gcacatgacg gaggttgtga ggcgctgccc ccaccatgag    540
   cgctgctcag atagcgatgg tctggcccct cctcagcatc ttatccgagt ggaaggaaat    600
   ttgcgtgtgg agtatttgga tgacagaaac acttttcgac atagtgtggt ggtgccctat    660
   gagccgcctg aggttggctc tgactgtacc accatccact acaactacat gtgtaacagt    720
   tcctgcatgg gcggcatgaa ccggaggccc atcctcacca tcatcacact ggaagactcc    780
   agtggtaatc tactgggacg gaacagcttt gaggtgcgtg tttgtgcctg tcctgggaga    840
   gaccggcgca cagaggaaga gaatctccgc aagaaagggg agcctcacca cgagctgccc    900
   ccagggagca ctaagcgagc actgcccaac aacaccagct cctctcccca gccaaagaag    960
   aaaccactgg atggagaata tttcaccctt cagatccgtg ggcgtgagcg cttcgagatg   1020
   ttccgagagc tgaatgaggc cttggaactc aaggatgccc aggctgggaa ggagccaggg   1080
   gggagcaggg ctcactccag ccacctgaag tccaaaaagg gtcagtctac ctcccgccat   1140
   aaaaaactca tgttcaagac agaagggcct gactcagac                          1179

## Beschreibung

Die vorliegende Erfindung betrifft Fusionsmoleküle von Antigenen, die dafür kodierenden Nukleinsäuren und die Verwendung derartiger Fusionsmolekül und Nukleinsäuren.

Erfindungsgemäße Fusionsmoleküle sind für eine Vielzahl von Anwendungen verwendbar, einschließlich zur Induktion einer Immunreaktion in einem Säuger.

Antigen-spezifische T-Zell-Reaktionen werden durch antigene Peptide, die an die Bindungsgrube von Glykoproteinen des Haupt-Histokompatibilitätskomplexes (MHC) gebunden sind, als Teil des Mechanismus des Immunsystems hervorgerufen, bei dem fremde Antigene identifiziert und eine Reaktion gegen sie ausgelöst wird. Die gebundenen antigenen Peptide interagieren mit T-Zell-Rezeptoren und modulieren so eine Immunreaktion. Die antigenen Peptide sind nicht-kovalent an bestimmte "Bindetaschen" gebunden, die von polymorphen Resten der Bindungsgrube des MHC-Proteins gebildet werden.

MHC Klasse II-Moleküle sind heterodimere Glykoproteine, die aus α- und β-Ketten bestehen. Die α1- und β1-Domänen dieser Moleküle falten sich zusammen und bilden eine Peptid-Bindungsgrube. Antigene Peptide binden an das MHC-Molekül durch Interaktion zwischen Anker-Aminosäuren auf dem Peptid und den α1- und β1-Domänen. Die Kristallstruktur des menschlichen Klasse II-HLA-DR1-Komplexes mit einem Influenzavirus-Peptid zeigt, dass sich die N- und C-terminalen Enden des gebundenen Peptids aus der Bindungsgrube erstrecken, so dass der C-Terminus des Peptids nahe zum N-Terminus der β-Kette liegt [Brown, J.H. et al., 1993, Nature 364:33-39; Stern, L.J. et al., 1994, Nature 368:215-221]. MHC Klasse I-Moleküle besitzen andere Domänen-Organisationen als MHC Klasse II-Moleküle, jedoch im Allgemeinen eine ähnliche Struktur mit einer Peptid-Bindestelle oder -grube, die zu den Membrandomänen entfernt liegt [vgl. z.B. Rudensky, A.Y. et al., 1991, Nature 353:622-627].

Der anfängliche Schritt bei der Präsentation eines fremden Protein-Antigens ist die Bindung des nativen Antigens an eine Antigen-präsentierende Zelle (APC). Nach Bindung an APCs dringen Antigene in die Zellen ein, entweder durch Phagozytose, Rezeptor-vermittelte Endozytose oder Pinozytose. Derartige internalisierte Antigene sind in intrazellulären Membrangebundenen Vesikeln, die Endosome genannt werden, lokalisiert. Nach einer Endosomen-Lysosomen-Fusion werden die Antigene in kleine Peptide durch in den Lysosomen gelegene zelluläre Proteasen prozessiert. Die Peptide assoziieren mit den α- und β-Ketten von MHC Klasse II-Molekülen innerhalb dieser Lysosomen. Diese MHC Klasse II-Moleküle, die zuvor im rauen endoplasmatischen Retikulum synthetisiert worden waren, werden sequentiell an die Golgi-Komplexe und sodann an das lysosomale Kompartiment transportiert. Der Peptid-MHC-Komplex wird auf der Oberfläche von APCs für eine T- und B-Zell-Aktivierung präsentiert. Daher sind die Zugängigkeit von proteolytischen Prozessierungsstellen in dem Antigen, die Stabilität der resultierenden Peptide in den Lysosomen und die Affinitäten der Peptide für MHC-Moleküle bestimmende Faktoren für die Immunogenität eines spezifischen Epitops.

Rekombinante Impfstoffe haben in der Human- und Veterinärmedizin eine besondere Bedeutung als Wirkstoffe und Arzneimittel für die Prophylaxe und Therapie von Infektions- und Krebserkrankungen. Ziel einer Impfung mit einem rekombinanten Impfstoff ist, gegen ein definiertes Antigen eine spezifische Immunreaktion zu induzieren, die präventiv oder therapeutisch gegen definierte Krankheiten wirksam ist.

Ein für die Effektivität eines rekombinanten Impfstoffs wesentlicher Faktor ist die optimale Stimulation von T-Lymphozyten des immunisierten Organismus. So belegt eine Reihe von tierexperimentellen Untersuchungen, dass für eine effektive Immuntherapie von Tumoren sowohl die optimale Stimulation von CD8⁺- als auch CD4⁺-Lymphozyten notwendig ist. Die bekannten Hauptformen von rekombinanten Vakzinen basieren auf rekombinanten Proteinen, synthetischen Peptidfragmenten, rekombinanten Viren und Nukleinsäureimpfstoffen auf DNA- bzw. RNA-Basis. In den letzten Jahren bekamen Impfstoffe auf DNA- und RNA-Nukleinsäurebasis eine zunehmende Bedeutung. Für sehr viele Ziele, unter anderem Tumorantigene, lässt sich jedoch mit rekombinanten Impfstoffen auf Nukleinsäurebasis eine nur sehr schlechte bis gar keine Stimulation von CD4⁺-Lymphozyten erreichen. Daher wurde eine Reihe gentechnischer Modifikationen entwickelt, mit der Absicht, die Immunogenität von rekombinanten Impfstoffen zu erhöhen. Hierbei sind bisher verschiedene Verfahren getestet worden, u.a. Verfremdung von Immunogenen durch Veränderung der Primärsequenz oder durch Fusion an Fremdepitope z.B. von Bakterien oder Viren [Lowenadler, B. et al., 1990, Eur. J. Immunol. 20: 1541-45; Clarke, B. E. et al., 1987, Nature 330: 381-84] und Herstellung von chimären Produkten, bestehend aus dem eigentlichen Antigen und immunmodulatorischen Proteinen wie z.B. Zytokinen [Ruckert, R. et al., 1998, Eur. J. Immunol. 28: 3312-20; Harvill, E. T., J. M. Fleming, und S. L. Morrison, 1996, J. Immunol. 157: 3165-70]. Impfstoffe, die auf Verfremdung basieren, induzieren zwar verstärkte Immunantworten, haben jedoch den großen Nachteil, dass die Immunstimulation gegen das Fremdepitop dominiert und dass Immunantworten gegen das eigentliche Vakzinetarget z.T. nur moderat bleiben.

Eine weitere attraktive Möglichkeit ist die Fusion mit Sequenzen von Proteinen, die eine Translokation des Proteins in degradierende Zellkompartimente erlauben sollen. Diese Modifikationen führen jedoch, wie mittlerweile bekannt, nur zu einer mäßig verbesserten Stimulation von CD4⁺-Lymphozyten und kaum zu einer Verstärkung von CD8⁺-Immunantworten [Wu, T. C. et al., 1995, Proc. Natl. Acad. Sci. U.S.A. 92: 11671-11675; Bonini, C. et al., 2001, J. Immunol. 166: 5250-57, Su, Z. et al., 2002, Cancer Res. 62: 5041-5048].

Somit wäre es wünschenswert, über Impfstoffe zu verfügen, die die Antigenpräsentation und damit die Immunogenität gegen ein bestimmtes Antigen deutlich erhöhen. Es wäre weiterhin wünschenswert, Impfstoffe systematisch so modifizieren zu können, dass eine maximale Immunantwort durch CD4⁺- und CD8⁺-Lymphozyten resultiert, ohne Fremdepitope einführen zu müssen.

Diese Aufgabe wird erfindungsgemäß durch den Gegenstand der Patentansprüche gelöst.

Erfindungsgemäß konnte festgestellt werden, dass Fusionsmoleküle, die Antigenmoleküle und Teile von Histokompatibilitätsantigenen umfassen, bei einer Verwendung als Impfstoffe eine um > 100-fach gesteigerte Immunogenität gegenüber den unmodifizierten Antigenen aufweisen und dass überraschenderweise sowohl Immunantworten von CD4+- als auch CD8+-T-Lymphozyten in bisher noch nicht beschriebener Weise erhöht werden.

Die vorliegende Erfindung betrifft allgemein Fusionsmoleküle von Antigenmolekülen und die Verwendung derartiger Fusionsmoleküle.

In einem Aspekt betrifft die Erfindung ein Fusionsmolekül, das ein Antigen, eine Transmembranregion und die cytoplasmatische Region einer Kette eines MHC-Moleküls umfasst, wobei das Fusionsmolekül keines der folgenden umfasst: (i) α1-, α2-, und α3-Domänen der α-Kette eines MHC-Klasse 1-Moleküls, (ii) β2-Mikroglobulin eines MHC-Klasse I-Moleküls, (iii) α1- und α2-Domänen der α-Kette eines MHC-Klasse II-Moleküls, und (iv) β1- und β2-Domänen der β-Kette eines MHC-Klasse II-Moleküls. Vorzugsweise sind sowohl die Transmembranregion als auch die cytoplasmatische Region von einem MHC-Molekül abgeleitet.

Somit wird insbesondere ein Fusionsmolekül bereitgestellt, das ein Antigen und den Teil einer Kette eines MHC-Moleküls umfasst, der im Wesentlichen der Sequenz der Transmembranregion in Verbindung mit der cytoplasmatischen Region eines MHC-Moleküls entspricht, wobei der Begriff "Transmembranregion in Verbindung mit der cytoplasmatischen Region" den Abschnitt einer Kette eines MHC-Moleküls betrifft, der mit dem N-terminalen Ende der Transmembranregion beginnt und mit dem C-terminalen Ende der cytoplasmatischen Region, insbesondere dem C-terminalen Ende der gesamten Kette des MHC-Moleküls abschließt. In dieser Ausführungsform entspricht die Verbindung der Transmembranregion mit der cytoplasmatischen Region der natürlich auftretenden Verbindung zwischen diesen Regionen.

Insbesondere wird erfindungsgemäß ein Fusionsmolekül bereitgestellt, das ein Antigen und einen Teil eine Kette eines MHC-Moleküls umfasst, wobei bei dem Teil im Wesentlichen die gesamten N-terminalen extrazellulären Domänen des MHC-Moleküls fehlen.

In einer besonders bevorzugten Ausführungsform bestehen die erfindungsgemäßen Fusionsmoleküle aus einer Fusion eines Antigens, gegebenenfalls mit einer Leitsequenz an seinem N-terminalen Ende, mit einer Transmembranregion, vorzugsweise einer Transmembranregion einer Kette eines MHC-Moleküls, am C-terminalen Ende des Antigens und einer cytoplasmatischen Region einer Kette eines MHC-Moleküls am C-terminalen Ende der Transmembranregion.

In einer besonders bevorzugten Ausführungsform umfassen die erfindungsgemäßen Fusionsmoleküle eine Leitsequenz, vorzugsweise eine Peptidsequenz mit Eigenschaften eines Sekretionssignals, das insbesondere in der Lage ist, die Translokation eines Proteins oder Peptids durch eine Membran zu steuern. Als Leitsequenz kann das Sekretionssignal jedes Typ-I Transmembranproteins genutzt werden, wobei der Begriff "Typ-I Transmembranprotein" solche Transmembranproteine betrifft, deren C-Terminus im Cytoplasma lokalisiert ist. In einer besonderes Ausführungsform ist die Leitsequenz von einer Kette eines MHC-Moleküls, abgeleitet. Vorzugsweise befindet sich die Leitsequenz am N-terminalen Ende der erfindungsgemäßen Fusionsmoleküle.

Insbesondere betrifft die Erfindung ein Fusionsmolekül, wobei im Wesentlichen die gesamten N-terminalen extrazellulären Domänen eines MHC-Moleküls durch ein Antigen mit einer Leitsequenz an dessen N-terminalen Ende ersetzt sind.

In einem erfindungsgemäßen Fusionsmolekül ist vorzugsweise das Antigen an seinem N-Terminus kovalent mit dem C-Terminus einer Leitsequenz verbunden und der C-Terminus des Antigenmoleküls ist mit dem N-Terminus der Transmembranregion verbunden, die ihrerseits am C-Terminus mit dem N-Terminus der cytoplasmatischen Region eines MHC-Moleküls verbunden ist.

Somit weist das erfindungsgemäße Fusionsmolekül vorzugsweise folgende Anordnung auf: N-Terminus-Leitsequenz/Antigen/Transmembranregion / cytoplasmatische Region-C-Terminus.

In einer besonders bevorzugten Ausführungsform besteht das erfindungsgemäße Fusionsmolekül im Wesentlichen aus der Leitsequenz, dem Antigen, der Transmembranregion und der cytoplasmatischen Region.

In einer besonders bevorzugten Ausführungsform ist das Antigen ein Peptid, Polypeptid oder Protein und das erfindungsgemäße Fusionsmolekül ist ein Protein oder Polypeptid.

In einer Ausführungsform sind mehrere Antigene, die gleich oder verschieden sein können, in dem erfindungsgemäßen Fusionsmolekül vorhanden, d.h. mindestens 2, vorzugsweise 2 bis 10, mehr bevorzugt 2 bis 5, noch mehr bevorzugt 2 bis 3, insbesondere 2 Antigene. Diese mehrfach gekoppelten Antigene können getrennt voneinander oder in Serie nacheinander, gegebenenfalls durch einen Linker getrennt, als Tandemkonstrukte vorliegen. Vorzugsweise wird dadurch bei Verabreichung eine Immunreaktion gegen verschiedene Antigene ausgelöst.

Das Antigen kann vollständig oder verkürzt sein, d.h. es enthält nur einen Teil des natürlichen Proteins oder Polypeptids, das als Antigen dient.

Vorzugsweise sind die Leitsequenz und/oder die Transmembranregion der erfindungsgemäßen Fusionsmoleküle von MHC-Molekülen, insbesondere der Klasse I oder II abgeleitet. Mehr bevorzugt sind die Leitsequenz und/oder die Transmembranregion und/oder die cytoplasmatische Region der erfindungsgemäßen Fusionsmoleküle von MHC-Molekülen, insbesondere der Klasse I oder II abgeleitet.

Erfindungsgemäß können auch eine oder mehrere, vorzugsweise flexible Linkersequenzen (Verbindungssequenzen) in dem Fusionsmolekül vorhanden sein, die zwischen der Leitsequenz und dem Antigen, zwischen dem Antigen und der Transmembranregion und/oder zwischen der Transmembranregion und der cytoplasmatischen Region liegen können. Vorzugsweise umfasst erfindungsgemäß eine Linkersequenz etwa 7 bis 20 Aminosäuren, besser etwa 8 bis 16 Aminosäuren, und insbesondere etwa 8 bis 12 Aminosäuren.

In erfindungsgemäßen Fusionsmolekülen ist die Linkersequenz vorzugsweise flexibel und hält so das damit verbundene Peptid nicht in einer einzigen, ungewünschten Konformation. Der Linker umfasst vorzugsweise vor allem Aminosäuren mit kleinen Seitenketten wie Glycin, Alanin und Serin, um eine Flexibilität zu ermöglichen. Vorzugsweise enthält die Linkersequenz keinen Prolinrest, der die Flexibilität hemmen könnte.

In einer weiteren Ausführungsform sind die Leitsequenz, das Antigen, die Transmembranregion und/oder die cytoplasmatische Region direkt ohne einen Linker miteinander verbunden.

Die Leitsequenz weist vorzugsweise die in SEQ ID NO: 2 gezeigte Sequenz oder eine davon abgeleitete Sequenz auf bzw. wird durch die in SEQ ID NO: 1 gezeigte Sequenz oder eine davon abgeleitete Sequenz kodiert. Die Transmembran-cytoplasmatische Region weist vorzugsweise die in SEQ ID NO: 4 bzw. 6 gezeigte Sequenz oder eine davon abgeleitete Sequenz auf bzw. wird durch die in SEQ ID NO: 3 bzw. 5 gezeigte Sequenz oder eine davon abgeleitete Sequenz kodiert.

In weiteren bevorzugten Ausführungsformen ist die Transmembran-cytoplasmatische bzw. die ausschließlich cytoplasmatische Region von sequenzverwandten MHC-Molekülen (u.a. HLA-A, HLA-B, HLA-C, HLA-E, HLA-F, HLA-DRa, HLA-DRb, HLA-DQa, HLA-DQb, HLA-DPa, HLA-DPb, CD1a, CD1b, CD1c) abgeleitet. Bevorzugte Transmembran-cytoplasmatische Regionen weisen eine Sequenz auf, ausgewählt aus der Gruppe bestehend aus den in SEQ ID NO: 15, 17, 19, 21, 23, 25, 27, 29, 31, 33, 35, 37, 39, 41 dargestellten Sequenzen und davon abgeleiteten Sequenzen. In weiteren Ausführungsformen weisen die ausschließlich cytoplasmatischen Regionen eine Sequenz auf, ausgewählt aus der Gruppe bestehend aus den in SEQ ID NO: 16, 18, 20, 22, 24, 26, 28, 30, 32, 34, 36, 38, 40, 42 dargestellten Sequenzen und davon abgeleiteten Sequenzen. In weiteren Ausführungsformen ist auch die Verwendung von abgewandelten Sequenzen, z.B. modifizierten oder orthologen Sequenzen aus anderen Organismen, vorgesehen. Besonders bevorzugt sind dabei Sequenzen, die am C-terminalen Ende eine Homologie von mehr als 60% zu den in SEQ ID NO: 16, 18, 20, 22, 24, 26, 28, 30, 32, 34, 36, 38, 40, 42 gezeigten Sequenzen aufweisen.

In einer besonders bevorzugten Ausführungsform umfasst das erfindungsgemäße Fusionsmolekül die in SEQ ID NO: 12 bzw. 14 gezeigte Aminosäuresequenz oder eine davon abgeleitete Sequenz.

In einem weiteren Aspekt betrifft die Erfindung Nukleinsäuren , die für die oben beschriebenen Fusionsmoleküle kodieren und vorzugsweise diese Fusionsmoleküle exprimieren können. Im folgenden umfasst der Begriff "Nukleinsäure" auch die Derivate davon.

In einer besonders bevorzugten Ausführungsform umfasst die Nukleinsäure, die für ein erfindungsgemäßes Fusionsmolekül kodiert, die in SEQ ID NO: 11 bzw. 13 gezeigte Nukleinsäuresequenz oder eine davon abgeleitete Sequenz.

Die Erfindung betrifft auch Wirtszellen, die eine erfindungsgemäße Nukleinsäure enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Erfindung eine pharmazeutische Zusammensetzung, insbesondere eine Vakzine, die ein oder mehrere der erfindungsgemäßen Fusionsmoleküle und/oder eine oder mehrere der dafür kodierenden Nukleinsäuren und/oder eine oder mehrere der erfindungsgemäßen Wirtszellen umfasst.

In einem weiteren Aspekt wird erfindungsgemäß ein in vitro-Verfahren zur Erhöhung der Menge an MHC/Peptid-Komplexen in einer Zelle bereitgestellt, wobei das Verfahren die Bereitstellung eines erfindungsgemäßen Fusionsmoleküls oder einer dafür kodierenden Nukleinsäure für die Zelle umfasst. Die Erfindung betrifft auch eine Verwendung eines erfindungsgemäßen Fusionsmoleküls oder einer dafür kodierenden Nukleinsäure zur Herstellung einer pharmazeutischen Zusammensetzung zur Erhöhung der Menge an MHC/Peptid-Komplexen in einer Zelle. In einer bevorzugten Ausführungsform ist die Zelle eine Antigen-präsentierende Zelle, insbesondere eine dendritische Zelle, ein Monozyt oder ein Makrophage.

In einem weiteren Aspekt wird erfindungsgemäß ein in vitro-Verfahren zur Steigerung der Präsentation von Zelloberflächenmolekülen auf Zellen bereitgestellt, die in der Lage sind, Antigene zu präsentieren (wie B-Zellen und Makrophagen, im Allgemeinen "APC" genannt). Die Verstärkung der Antigen-präsentierenden Aktivität solcher Zellen erfolgt durch Bereitstellung eines erfindungsgemäßen Fusionsmoleküls oder einer dafür kodierenden Nukleinsäure für die Zellen. Die Erfindung betrifft auch eine Verwendung eines erfindungsgemäßen Fusionsmoleküls oder einer dafür kodierenden Nukleinsäure zur Herstellung einer pharmazeutischen Zusammensetzung zur Steigerung der Präsentation von Zelloberflächenmolekülen auf Zellen, die in der Lage sind, Antigene zu präsentieren, insbesondere B-Zellen und Makrophagen. Eine derartige Verstärkung der Antigen-präsentierenden Aktivität verstärkt wiederum vorzugsweise die primäre Aktivierung von T-Zellen, insbesondere von CD4⁺- und CD8⁺-Lymphozyten, die gegenüber dem Antigen reagieren.

In einem weiteren Aspekt wird erfindungsgemäß eine Verwendung eines erfindungsgemäßen Fusionsmoleküls und/oder einer dafür kodierenden Nukleinsäure und/oder einer erfindungsgemäßen Wirtszelle zur Herstellung einer pharmazeutischen Zusammensetzung zum Auslösen einer Immunreaktion bei einem Lebewesen bereitgestellt.

In einem weiteren Aspekt wird erfingdungsgemäß ein in vitro-Verfahren zur Stimulierung oder Aktivierung von T-Zellen, insbesondere CD4⁺- und CD8⁺-Lymphozyten, bereitgestellt, wobei das Verfahren die Bereitstellung für die T-Zellen eines erfindungsgemäßen Fusionsmoleküls und/ oder einer dafür kodierenden Nukleinsäure und / oder einer erfindungsgemäßen Wirtszelle umfasst. Die Erfindung betrifft auch eine Verwendung eines erfindungsgemäßen Fusionsmoleküls und/oder einer dafür kodierenden Nukleinsäure und/oder einer erfindungsgemäßen Wirtszelle zur Herstellung einer pharmazeutischen Zusammensetzung zur Stimulierung oder Aktivierung von T-Zellen, insbesondere CD4⁺- und CD8⁺-Lymphozyten, in einem Lebewesen, insbesondere einem Patienten. Eine derartige Stimulierung oder Aktivierung äußert sich vorzugsweise in einer Expansion, cytotoxischen Reaktivität und/ oder Zytokinausschüttung der T-Zellen.

In einem weiteren Aspekt wird eine Verwendung eines erfindungsgemäßen Fusionsmoleküls und/oder einer dafür kodierenden Nukleinsäure und/oder einer erfindungsgemäßen Wirtszelle zur Herstellung einer pharmazeutischen Zusammensetzung zur Behandlung, Vakzinierung oder Immunisierung eines Lebewesens bereitgestellt. Dabei werden insbesondere solche Antigene in dem erfindungsgemäßen Fusionsmolekül oder der dafür kodierenden Nukleinsäure eingesetzt, die ohne die erfindungsgemäße Veränderung als für die beabsichtigte Behandlung, Vakzinierung oder Immunisierung wirksam bekannt sind.

Die vorstehend beschriebenen Anseitze eignen sich insbesondere für eine Behandlung oder Prophylaxe von infektiösen Erkrankungen, die beispielsweise von Bakterien oder Viren verursacht werden. In bestimmten Ausführungsformen ist das erfindungsgemäß verwendete Antigen von einem infektiösen Erreger wie Hepatitis A, B, C, HIV, Mykobakterien, Malariaerreger, Erreger von SARS, Herpesvirus, Influenzavirus, Poliovirus bzw. von bakteriellen Erregern wie Chlamydien und Mykobakterien abgeleitet. Eine besonders nützliche Anwendung der vorliegenden Erfindung liegt in der Krebs-Immuntherapie oder -Vakzinierung, wo insbesondere eine Aktivierung von Tumorantigen-reaktiven T-Zellen verstärkt wird, wodurch die Aussicht für eine T-Zell-Immuntherapie oder -Vakzinierung gegen Tumorzellen verbessert wird.

In spezifischen Ausführungsformen ist das erfindungsgemäß verwendete Antigen ausgewählt aus der Gruppe bestehend aus den folgenden Antigenen: p53, vorzugsweise kodiert von der in SEQ ID NO: 45 gezeigten Sequenz, ART-4, BAGE, ss-Catenin/m, Bcr-abL CAMEL, CAP-1, CASP-8, CDC27/m, CDK4/m, CEA, CLAUDIN-12, c-MYC, CT, Cyp-B, DAM, ELF2M, ETV6-AML1, G250, GAGE, GnT-V, Gap100, HAGE, HER-2/neu, HPV-E7, HPV-E6, HAST-2, hTERT (oder hTRT), LAGE, LDLR/FUT, MAGE-A, vorzugsweise MAGE-A1, MAGE-A2, MAGE-A3, MAGE-A4, MAGE-A5, MAGE-A6, MAGE-A7, MAGE-A8, MAGE-A9, MAGE-A10, MAGE-A11 oder MAGE-A12, MAGE-B, MAGE-C, MART- 1/Melan-A, MC1R, Myosin/m, MUC1, MUM-1, -2, -3, NA88-A, NF1, NY-ESO-1, NY-BR-1, p190 minor bcr-abL Pml/RARa, PRAME, Proteinase-3, PSA, PSM, RAGE, RU1 oder RU2, SAGE, SART-1 oder SART-3, SCGB3A2, SCP1, SCP2, SCP3, SSX, SURVIVIN, TEL/AML1, TPI/m, TRP-1, TRP-2, TRP-2/INT2, TPTE und WT, vorzugsweise WT-1, insbesondere kodiert von der in SEQ ID NO: 44 gezeigten Sequenz.

### Detaillierte Beschreibung der Erfindung

Die Begriffe "Domäne" oder "Region" betreffen einen bestimmten Teil einer Aminosäuresequenz, der vorzugsweise mit einer bestimmten Funktion oder Struktur in Zusammenhang gebracht werden kann. Zum Beispiel weisen die α- und β-Polypeptide eines MHC-Klasse II-Moleküls zwei Domänen, α1, α2 bzw. β1, β2, eine Transmembranregion und eine cytoplasmatische Region auf. In ähnlicher Weise weist die α-Kette von MHC-Klasse I-Molekülen drei Domänen, α1, α2 und α3, eine Transmembranregion und eine cytoplasmatische Region auf.

In einer Ausführungsform wird bei einer Auswahl der Sequenz einer bestimmten Domäne oder Region für eine Deletion oder einen Einbau in ein erfindungsgemäßes Fusionsmolekül die gesamte Domäne oder Region umfasst. Um dieses sicherzustellen, kann die Sequenz der betreffenden Domäne oder Region verlängert sein, um Teile eines Linkers oder sogar Teile der benachbarten Domäne oder Region zu beinhalten. Der Begriff "im Wesentlichen" in Bezug auf eine Domäne oder Region ist in diesem Sinne zu verstehen.

Der Begriff "Transmembranregion" betrifft den Teil eines Proteins, der im Wesentlichen den sich in einer zellulären Membran befindlichen Anteil ausmacht und vorzugsweise einer Verankerung des Proteins in der Membran dient. Vorzugsweise ist erfindungsgemäß eine Transmembranregion eine Aminosäuresequenz mit einem einzelnen Durchtritt durch die Membran. Jedoch kann in bestimmten Ausführungsformen auch eine Transmembranregion verwendet werden, die mehr als einen Durchtritt durch die Membran aufweist. Die Transmembranregion wird im Allgemeinen 15-25 vorzugsweise hydrophobe ungeladene Aminosäuren aufweisen, die beispielsweise eine α-helikale Konformation einnehmen. Vorzugsweise ist die Transmembranregion von einem Protein, ausgewählt aus der Gruppe bestehend aus MHC-Molekülen, Immunglobulinen, CD4, CD8, der CD3-ξ-Kette, der CD3-γ-Kette, der CD3-δ-Kette und der CD3-ε-Kette abgeleitet.

Typischerweise besteht die Transmembranregion im Fall der α- und β-Ketten des MHC Klasse II-Moleküls aus etwa 20 hydrophoben Aminosäuren, die mit dem Carboxy-terminalen Ende des Antigens verbunden sind. Diese Reste erlauben ein Überspannen der Membran durch das Protein. Die Transmembranregion endet mit etwa 6-32 Resten, die den cytoplasmatischen Schwanz umfassen, am Carboxy-terminalen Ende einer jeden dieser Ketten. Es wurde gezeigt, dass diese Transmembran- und cytoplasmatischen Regionen durch Sequenzen ersetzt werden können, die eine GPI-Bindung signalisieren, und dass die chimären GPI-verankerten Klasse II-Moleküle Membran-gebunden sind (Wettstein, D.A., J.J. Boniface, P.A. Reay, H. Schild und M.M. Davis, 1991, J. Exp. Med. 174:219-228). Solche Ausführungsformen sind erfindungsgemäß von dem Begriff "Transmembranregion" umfasst. GPI-gebundene Membran-Ankerdomänen wurden in einer Reihe von Proteinen definiert, einschließlich Verfallbeschleunigender Faktor (decay accelerating factor; DAF), CD59 und menschlicher placentaler alkalischer Phosphatase (HPAP) (Wettstein, D.A., J.J. et al., 1991, J. Exp. Med. 174:219-228). Zum Beispiel sind die 38 Carboxy-terminalen Aminosäuren von HPAP ausreichend für eine Funktion als Signalsequenz für eine Bindung von GPI. Falls die für diese Domäne kodierende DNA-Sequenz mit einem sekretierten Molekül, wie dem löslichen Teil der MHC Klasse II-α- oder -β-Kette verbunden wird, bildet sich ein Membran-gebundenes chimäres Molekül (Wettstein, D.A. et al., 1991, J. Exp. Med. 174:219-228) und ein derartiges Verfahren kann für die Verankerung von Fusionsmolekülen der Erfindung an eine Zellmembran eingesetzt werden.

Der Begriff "Haupt-Histokompatibilitätskomplex" und die Abkürzung "MHC" betreffen einen Komplex von Genen, der in allen Vertebraten auftritt. MHC-Proteine oder -Moleküle fungieren bei einer Signalgebung zwischen Lymphozyten und Antigen-präsentierenden Zellen in normalen Immunreaktionen dadurch, dass sie Peptide binden und sie für eine mögliche Erkennung durch T-Zell-Rezeptoren (TCR) präsentieren. MHC-Moleküle binden Peptide in einem intrazellulären Prozessierungskompartiment und präsentieren diese Peptide auf der Oberfläche von Antigen-präsentierenden Zellen gegenüber T-Zellen. Die menschliche MHC-Region, auch als HLA bezeichnet, findet sich auf Chromosom 6 und beinhaltet die Klasse I-Region und die Klasse II-Region.

Der Begriff "MHC-Klasse I" oder "Klasse I" betrifft die Haupt-Histokompatibilitätskomplex-Klasse I-Proteine oder -Gene. Innerhalb der MHC-Klasse I-Region finden sich beim Menschen die HLA-A-, HLA-B-, HLA-C-, HLA-E-, HLA-F-, CD1a-, CD1b- und CD1c-Unterregionen.

Die α-Ketten der Klasse I sind Glykoproteine mit einem Molekulargewicht von etwa 44 kDa. Die Polypeptidkette ist etwas über 350 Aminosäurereste lang. Sie kann in drei funktionelle Regionen unterteilt werden: eine externe, eine transmembranöse und eine cytoplasmatische Region. Die externe Region ist 283 Aminosäurereste lang und in drei Domänen, α1, α2 und α3, unterteilt. Die Domänen und Regionen werden gewöhnlich von separaten Exons des Klasse I-Gens kodiert. Die transmembrane Region umspannt die Lipid-Doppelschicht der Plasmamembran. Sie besteht aus 23 zumeist hydrophoben Aminosäureresten, die in einer α-Helix angeordnet sind. Die cytoplasmatische Region, d.h. der dem Cytoplasma zugewandte Teil, der sich an die Transmembranregion anschließt, ist typischerweise 32 Aminosäurereste lang und hat die Fähigkeit, mit den Elementen des Cytoskeletts zu interagieren. Die α-Kette interagiert mit β2-Mikroglobulin und bildet so α-β2-Dimere auf der Zelloberfläche.

Der Begriff "MHC-Klasse II" oder "Klasse II" betrifft die Haupt-Histokompatibilitätskomplex-Klasse II-Proteine oder -Gene. Innerhalb der MHC-Klasse II-Region finden sich im Menschen die DP-, DQ- und DR-Subregionen für Klasse II-α-Ketten- und -β-Ketten-Gene (d.h. DPα, DPβ, DQα, DQβ, DRα und DRβ).

Klasse II-Moleküle sind Heterodimere, die aus je einer α- und einer β-Kette bestehen. Beide Ketten sind Glykoproteine mit einem Molekulargewicht von 31-34 kDa (α) oder 26-29 kDa (β). Die Gesamtlänge der α-Ketten variiert von 229 bis 233 Aminosäureresten, die der β-Ketten von 225 bis 238 Resten. Sowohl α- als auch β-Ketten bestehen aus einer externen Region, einem verbindenden Peptid, einer transmembranösen Region und einem cytoplasmatischen Schwanz. Die externe Region besteht aus zwei Domänen, α1 und α2 oder β1 und β2. Das verbindende Peptid ist in α- und β-Ketten 13 bzw. 9 Reste lang. Es verbindet die zweite Domäne mit der transmembranösen Region, die sowohl in α- als auch in β-Ketten aus 23 Aminosäureresten besteht. Die Länge der cytoplasmatischen Region, d.h. der dem Cytoplasma zugewandte Teil, der sich an die Transmembranregion anschließt, variiert von 3 bis 16 Resten in α-Ketten und von 8 bis 20 Resten in β-Ketten.

Erfindungsgemäß betrifft der Begriff "Kette eines MHC-Moleküls" die α-Kette eines MHC-Klasse 1-Moleküls bzw. die α- und β-Ketten eines MHC-Klasse II-Moleküls. Die α-Ketten eines MHC-Klasse I-Moleküls, von denen die erfindungsgemäßen Fusionsmoleküle abgeleitet sein können, umfassen die HLA-A-, -B- und -C-α-Ketten. Die α-Ketten eines MHC-Klasse II-Moleküls, von denen die erfindungsgemäßen Fusionsmoleküle abgeleitet sein können, umfassen HLA-DR-, -DP- und -DQ-α-Ketten, insbesondere HLA-DR1-, HLA-DR2-, HLA-DR4-, HLA-DQ1-, HLA-DQ2- und HLA-DQ8-α-Ketten und insbesondere α-Ketten, die von DRA*0101-, DRA*0102-, DQA1*0301- oder DQA1*0501-Allelen kodiert werden. Die β-Ketten eines MHC-Klasse II-Moleküls, von denen die erfindungsgemäßen Fusionsmoleküle abgeleitet sein können, umfassen HLA-DR-, -DP- und -DQ-β-Ketten, insbesondere HLA-DR1-, HLA-DR2-, HLA-DR4-, HLA-DQ1-, HLA-DQ2- und HLA-DQ8-β-Ketten und insbesondere β-Ketten, die von DRB1*01-, DRB1*15-, DRB1*16-, DRBS*01-, DQB1*03- und DQB1*02-Allelen kodiert werden.

Der Begriff, "MHC-Bindedomäne" betrifft die "MHC-Klasse I-Bindedomäne" und "MHC-Klasse II-Bindedomäne".

Der Begriff "MHC-Klasse I-Bindedomäne" betrifft die Region eines MHC-Klasse I-Moleküls oder einer MHC-Klasse I-Kette, die für eine Bindung an ein antigenes Peptid notwendig ist. Eine MHC-Klasse I-Bindedomäne wird vorwiegend durch die α1- und α2-Domänen der MHC-Klasse I-α-Kette gebildet. Obwohl die α3-Domäne der α-Kette und β2-Mikroglobulin keine essentiellen Teile der Bindedomäne darstellen, sind sie vermutlich für eine Stabilisierung der Gesamtstruktur des MHC-Klasse I-Moleküls wichtig und daher schließt der Begriff "MHC-Klasse I-Bindedomäne" diese Regionen vorzugsweise ein. Eine MHC-Klasse I-Bindedomäne kann auch im Wesentlichen als die extrazelluläre Domäne eines MHC-Klasse I-Moleküls definiert werden, was sie von den Transmembran- und cytoplasmatischen Regionen unterscheidet.

Der Begriff "MHC-Klasse II-Bindedomäne" betrifft die Region eines MHC-Klasse II-Moleküls oder einer MHC-Klasse II-Kette, die für die Bindung an ein antigenes Peptid notwendig ist. Eine MHC-Klasse II-Bindedomäne wird vorwiegend durch die α1- und β1-Domänen der MHC-Klasse II-α- und -β-Ketten gebildet. Die α2- und β2-Domänen dieser Proteine sind vermutlich jedoch auch für eine Stabilisierung der Gesamtstruktur der MHC-Bindegrube wichtig und daher schließt der Begriff "MHC-Klasse II-Bindedomäne" erfindungsgemäß vorzugsweise diese Regionen ein. Eine MHC-Klasse II-Bindedomäne kann auch im Wesentlichen als die extrazelluläre Domäne eines MHC-Klasse II-Moleküls definiert werden, was sie von der Transmembran- und cytoplasmatischen Domäne unterscheidet.

Die genaue Anzahl an Aminosäuren in den verschiedenen MHC-Moleküldomänen oder -Regionen variiert abhängig von der Säugerspezies sowie zwischen Genklassen innerhalb einer Spezies. Bei einer Auswahl der Aminosäuresequenz einer bestimmten Domäne oder Region ist vielmehr die Aufrechterhaltung der Funktion der Domäne oder Region wichtig als die genaue strukturelle Definition, die auf der Anzahl von Aminosäuren basiert. Ferner ist dem Fachmann bekannt, dass die Funktion auch aufrechterhalten werden kann, wenn etwas weniger als die gesamte Aminosäuresequenz der ausgewählten Domäne oder Region verwendet wird.

Der Begriff "Antigen" betrifft ein Agens, gegen das eine Immunreaktion erzeugt werden soll. Der Begriff "Antigen" umfasst insbesondere Proteine, Peptide, Polysaccharide, Nukleinsäuren insbesondere RNA und DNA sowie Nukleotide. Der Begriff "Antigen" umfasst auch derivatisierte Antigene als erst durch Umwandlung (z.B. intermediär im Molekül, durch Komplettierung mit Körpereiweiss) antigen werdende - und sensibilisierende - Sekundärsubstanz und konjugierte Antigene, die durch künstlichen Einbau von Atomgruppen (z.B. Isocyanate, Diazoniumsalze) eine neue konstitutive Spezifität aufweisen. In einer bevorzugten Ausführungsform ist das Antigen ein Tumorantigen, d.h. ein Bestandteil von Krebszellen, die dem Cytoplasma, der Zelloberfläche und dem Zellkern entstammen können, insbesondere diejenigen, die intrazellulär oder als Oberflächenantigene an Tumorzellen vorzugsweise vermehrt entstehenden Antigene. Beispiele sind das karzinoembryonale Antigen, α1-Fetoprotein, Isoferritin und fetales Sulfoglykoprotein, α2-H-Ferroprotein und γ-Fetoprotein und verschiedene Virustumorantigene. In einer weiteren Ausführungsform ist das Antigen ein Virusantigen wie virale Ribonukleoproteine oder Hüllproteine. Insbesondere sollte das Antigen oder Peptide davon von MHC-Molekülen präsentiert werden und dadurch zur Modulation, insbesondere Aktivierung von Zellen des Immunsystems, vorzugsweise CD4⁺- und CD8⁺-Lymphozyten, insbesondere über die Modulation der Aktivität eines T-Zell-Rezeptors fähig sein und somit vorzugsweise die T-Zell-Vermehrung induzieren.

Der Begriff "MHC/Peptid-Komplex" betrifft einen nicht-kovalenten Komplex der Bindedomäne eines MHC-Klasse I- bzw. MHC-Klasse II-Moleküls und eines MHC-Klasse I- bzw. MHC-Klasse II-Bindepeptids.

Der Begriff "MHC-Bindepeptid" oder "bindendes Peptid" betrifft ein Peptid, das an ein MHC-Klasse I- und/oder ein MHC-Klasse II-Molekül bindet. Im Fall von Klasse I-MHC/Peptid-Komplexen sind die Bindepeptide typischerweise 8-10 Aminosäuren lang, obwohl längere oder kürzere Peptide wirksam sein können. Im Fall von Klasse II-MHC/Peptid-Komplexen sind die Bindepeptide typischerweise 10-25 Aminosäuren lang und insbesondere 13-18 Aminosäuren lang, obwohl längere und kürzere Peptide wirksam sein können.

Erfindungsgemäße Fusionsmoleküle und die dafür kodierenden Nukleinsäuren können im Allgemeinen durch rekombinante DNA-Techniken wie Herstellung von Plasmid-DNA, Spaltung von DNA mit Restriktionsenzymen, Ligation von DNA, Transformation oder Transfektion eines Wirts, Kultivierung des Wirts und Isolierung und Reinigung des exprimierten Fusionsmoleküls hergestellt werden. Solche Verfahren sind bekannt und z.B. in Sambrook et al., Molecular Cloning, (2. Auflage, 1989) beschrieben.

Für das Antigen kodierende DNA kann durch Isolation von DNA aus natürlichen Quellen oder durch bekannte Syntheseverfahren wie dem Phosphattriesterverfahren erhalten werden; vgl. z.B. Oligonucleotide Synthesis, IRL Press (M.J. Gait, Hrsg., 1984). Synthetische Oligonukleotide können auch mit Hilfe käuflich erhältlicher automatischer Oligonukleotid-Synthesegeräte hergestellt werden.

Die Anteile von MHC-Moleküle der erfindungsgemäßen Fusionsmoleküle entsprechen in Bezug auf die Aminosäuresequenz in geeigneter Weise natürlich vorkommenden MHC-Molekülen von Mensch, Maus oder anderen Nagern oder anderen Säugern oder sind Derivate davon.

Für MHC-Proteine kodierende DNA-Quellen sind bekannt, wie menschliche lymphoblastoide Zellen. Nach einer Isolierung kann das für das MHC-Molekül kodierende Gen oder ein interessierender Teil davon durch Polymerase-Kettenreaktion (PCR) oder andere bekannte Verfahren amplifiziert werden. Geeignete PCR-Primer für die Amplifikation des Gens für das MHC-Peptid können Restriktionsstellen an das PCR-Produkt anfügen.

Vorzugsweise werden erfindungsgemäß DNA-Konstrukte hergestellt, die für die Leitsequenz, die Transmembranregion und die cytoplasmatische Region kodierende Nukleinsäuresequenzen umfassen und die eine Restriktionsschnittstelle zwischen der Leitsequenz und der Transmembranregion enthalten, so dass im Wesentlichen jede für ein interessierendes Antigen kodierende Nukleotidsequenz in das Konstrukt eingebunden werden kann.

In einem bevorzugten Verfahren zur Herstellung erfindungsgemäßer Fusionsmoleküle werden DNA-Sequenzen so angeordnet, dass das C-terminale Ende der Leitsequenz an das N-terminale Ende des Antigens, das C-terminale Ende des Antigens an das N-terminale Ende der Transmembranregion, und das C-terminale Ende der Transmembranregion an das N-terminale Ende der cytoplasmatischen Region gebunden ist. Wie vorstehend erörtert, werden vorzugsweise Restriktionsschnittstellen zwischen das Ende der Leitsequenz und den Anfang der Transmembranregion eingebaut, so dass im Wesentlichen jede Nukleinsäure, die für ein interessierendes Antigen kodiert, an die Nukleinsäuresequenz für die Transmembranregion gebunden werden kann.

Ein exprimiertes erfindungsgemäßes Fusionsmolekül kann isoliert und in an sich bekannter Weise gereinigt werden. Typischerweise wird das Kulturmedium zentrifugiert und der Überstand sodann durch Affinitäts- oder Immunoaffinitätsverfahren, umfassend die Verwendung von monoklonalen Antikörpern, die an das exprimierte Fusionsmolekül binden, gereinigt. Das Fusionsmolekül kann auch eine Sequenz enthalten, die die Reinigung unterstützt, z.B. ein 6xHis-Tag.

Die Fähigkeit eines erfindungsgemäßen Fusionsmoleküls, die Aktivität eines T-Zell-Rezeptors zu modulieren (einschließlich Inaktivierung der T-Zell-Reaktionen) kann einfach durch einen in vitro-Test bestimmt werden. Typischerweise werden T-Zellen für die Tests durch transformierte T-Zelllinien bereitgestellt, wie T-Zell-Hybridome oder T-Zellen, die aus einem Säuger wie einem Menschen oder einem Nagetier wie einer Maus isoliert werden. Geeignete T-Zell-Hybridome sind frei verfügbar oder können in an sich bekannter Weise hergestellt werden. T-Zellen können in an sich bekannter Weise aus einem Säuger isoliert werden; vgl. z.B. Shimonkevitz, R. et al., 1983, J. Exp. Med. 158:303.

Ein geeigneter Test zur Bestimmung, ob ein erfindungsgemäßes Fusionsmolekül zur Modulation der Aktivität von T-Zellen fähig ist, erfolgt wie folgt durch die nachstehenden Schritte 1-4. T-Zellen exprimieren in geeigneter Weise einen Marker, der getestet werden kann und der T-Zell-Aktivierung oder Modulation der T-Zell-Aktivität nach Aktivierung anzeigt. So kann das Maus-T-Zell-Hybridom DO11.10, das Interleukin-2 (IL-2) bei einer Aktivierung exprimiert, verwendet werden. IL-2-Konzentrationen können gemessen werden, um zu bestimmen, ob ein spezifisches präsentierendes Peptid zur Modulation der Aktivität dieses T-Zell-Hybridoms fähig ist. Ein derartiger geeigneter Test erfolgt durch die nachstehenden Schritte:
1. T-Zellen werden z.B. aus einem interessierenden T-Zell-Hybridom oder durch Isolierung aus einem Säuger erhalten.
2. Die T-Zellen werden unter Bedingungen kultiviert, die eine Vermehrung erlauben.
3. Die wachsenden T-Zellen werden mit Antigen-präsentierenden Zellen in Kontakt gebracht, die ihrerseits mit einem erfindungsgemäßen Fusionsmolekül oder einer dafür kodierenden Nukleinsäure in Kontakt gebracht wurden.
4. Die T-Zellen werden auf einen Marker getestet, z.B. wird die IL-2-Produktion gemessen.

Die in den Tests verwendeten T-Zellen werden unter für eine Vermehrung geeigneten Bedingungen inkubiert. Zum Beispiel wird ein DO11.10-T-Zell-Hybridom geeigneterweise bei etwa 37°C und 5% CO₂ im Vollmedium (RPMI 1640, supplementiert mit 10% FBS, Penicillin/Streptomycin, L-Glutamin und 5 x 10⁻⁵ M 2-Mercaptoethanol) inkubiert. Serielle Verdünnungen des erfindungsgemäßen Fusionsmoleküls können getestet werden. T-Zell-Aktivierungssignale werden durch Antigen-präsentierende Zellen bereitgestellt, die mit dem geeigneten antigenen Peptid beladen worden waren.

Alternativ zu der Messung eines exprimierten Proteins wie IL-2 kann die Modulation der T-Zell-Aktivierung geeigneter Weise durch Veränderungen der Vermehrung von Antigen-abhängigen T-Zellen, wie gemessen durch bekannte Radiomarkierungsverfahren, bestimmt werden. Zum Beispiel kann ein markiertes (wie tritiert) Nukleotid in ein Testkulturmedium eingebracht werden. Das Einbringen eines derartigen markierten Nukleotids in die DNA dient als Messgröße für die T-Zell-Vermehrung. Dieser Test ist nicht für T-Zellen geeignet, die keiner Antigen-Präsentation für das Wachstum bedürfen, wie T-Zell-Hybridome. Der Test ist für die Messung der Modulation von T-Zell-Aktivierung durch Fusionsmoleküle im Fall von nicht-transformierten T-Zellen, die aus Säugern isoliert wurden, geeignet.

Die Fähigkeit eines erfindungsgemäßen Fusionsmoleküls, eine Immunreaktion zu induzieren, einschließlich eine Vakzinierung gegen eine Zielerkrankung zu ermöglichen, kann einfach durch einen in vivo-Test bestimmt werden. Zum Beispiel kann ein erfindungsgemäßes Fusionsmolekül oder eine dafür kodierende Nukleinsäure an einen Säuger wie eine Maus verabreicht werden und Blutproben aus dem Säuger zum Zeitpunkt der ersten Verabreichung und mehrfach in periodischen Zeiträumen danach (wie 1, 2, 5 und 8 Wochen nach Verabreichung des Fusionsmoleküls oder der dafür kodierenden Nukleinsäure) entnommen werden. Serum wird aus den Blutproben gewonnen und auf das Auftreten von durch die Immunisierung entstandenen Antikörpern getestet. Antikörper-Konzentrationen können bestimmt werden. Daneben können aus dem Blut bzw. aus lymphatischen Organen T-Lymphozyten isoliert werden und funktionell auf Reaktivitität gegen das Antigen bzw. von dem Antigen abgeleitete Epitope getestet werden. Alle dem Fachmann bekannten "Readout"-Systeme u.a. Proliferationsassay, Zytokinsekretion, zytotoxische Aktivität, Tetrameranalyse können hierbei verwendet werden.

Erfindungsgemäß Kann die Induktion einer Immunreaktion, einschließlich der Vakzinierung eines Lebewesens gegen eine Zielerkrankung, in Kombination mit bekannten Miglichkeiten für die Induktion einer Immunreaktion erfalgen. Zum Beispiel kann ein erfindungsgemäßes Fusionsmolekül oder eine dafür kodierende Nukleinsäure an ein Lebewesen in einer Anordnung oder Kombination mit der Verabreichung einer Vakzine-Zusammensetzung verabreicht werden, um die gewünschte Wirkung einer derartigen Vakzine-Zusammensetzung zu verstärken oder zu verlängern.

Der Begriff "abgeleitet" bedeutet erfindungsgemäß, dass ein bestimmter Gegenstand, insbesondere eine bestimmte Sequenz, in dem Objekt, von dem er abgeleitet ist, insbesondere einem Organismus oder Molekül, vorliegt. Im Fall von Nukleinsäure- und Aminosäuresequenzen, insbesondere bestimmten Sequenzregionen, bedeutet "abgeleitet" außerdem, dass die betreffende Nukleinsäure- bzw. Aminosäuresequenz in Übereinstimmung mit den nachstehenden Definitionen von einer Nukleinsäure- bzw. Aminosäuresequenz abgeleitet ist, die in dem Objekt vorliegt. Somit bedeutet der Begriff "von einem MHC-Molekül abgeleitete Sequenz oder Region", dass die Sequenz oder Region in einem MHC-Molekül vorliegt oder von einer Sequenz oder Region, die in einem MHC-Molekül vorliegt, in Übereinstimmung mit den nachstehenden Definitionen abgeleitet ist.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfingdungsgemäß als einzelsträngiges oder doppelsträngiges und lineares oder kovalent kreisförmig geschlossenes Molekül vorliegen.

Eine von einer Nukleinsäuresequenz abgeleitete Sequenz oder der Begriff "von einer Nukleinsäuresequenz abgeleitete Sequenz" betrifft erfindungsgemäß homologe Sequenzen und Derivate der ersteren Sequenz.

Homologe Nukleinsäuresequenzen weisen erfindungsgemäß mindestens 40%, insbesondere mindestens 50%, mindestens 60%, mindestens 70%, mindestens 80%, mindestens 90% und vorzugsweise mindestens 95%, mindestens 98 oder mindestens 99% Identität der Nukleotide auf.

Eine Nukleinsäure ist insbesondere dann zu einer anderen Nukleinsäure "homolog", wenn die beiden Sequenzen der komplementären Stränge miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York, beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH 7), 0,5% SDS, 2mM EDTA). SSC ist 0,15 M Natriumchlorid / 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) in vitro amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Nukleinsäuren, die für Fusionsmoleküle kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im Allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch Enhancer-Sequenzen oder stromaufwärts gelegene Aktivatorsequenzen umfassen.

In einer bevorzugten Ausführungsform ist die Nukleinsäure erfindungsgemäß ein Vektor, gegebenenfalls mit einem Promotor, der die Expression einer Nukleinsäure, z.B. einer Nukleinsäure, die für ein erfindungsgemäßes Fusionsmolekül kodiert, steuert. In einer bevorzugten Ausführungsform ist der Promoter ein T7-, T3- oder SP6-Promoter.

Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide, Bacteriophage oder Virusgenome.

Die Nukleinsäuren, die für ein erfindungsgemäßes Fusionsmolekül kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. E. coli) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Prirnaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

Eine für ein erfindungsgemäßes Fusionsmolekül kodierende Nukleinsäure kann auch eine Nukleinsäuresequenz umfassen, die für ein MHC-Molekül, vorzugsweise für ein HLA-Molekül kodiert. Die Nukleinsäuresequenz, die für ein MHC-Molekül kodiert, kann auf demselben Expressionsvektor wie die Nukleinsäure, die für das Fusionsmolekül kodiert, vorliegen oder beide Nukleinsäuren können auf verschiedenen Expressionsvektoren vorliegen. Im letzteren Fall können die beiden Expressionsvektoren in eine Zelle cotransfiziert werden.

Eine von einer Aminosäuresequenz abgeleitete Sequenz oder der Begriff "von einer Aminosäuresequenz abgeleitete Sequenz" betrifft erfindungsgemäß homologe Sequenzen und Derivate der ersteren Sequenz.

Homologe Aminosäuresequenzen weisen erfindungsgemäß mindestens 40%, insbesondere mindestens 50%, mindestens 60%, mindestens 70%, mindestens 80%, mindestens 90% und vorzugsweise mindestens 95%, mindestens 98 oder mindestens 99% Identität der Aminosäurereste auf.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die vorstehend beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinanten DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen und die allgemeinen rekombinanten Verfahren zur Expression von Proteinen z.B. in einem biologischen System (wie Säuger-, Insekten-, Pflanzen- und viralen Systeme) sind z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Protein oder Polypeptid assoziiert sind, wie Kohlenhydrate, Lipide und/oder Proteine oder Polypeptide.

In einer Ausführungsform umfassen "Derivate" von Proteinen oder Polypeptiden diejenigen modifizierten Analoga, die durch Glykosylierung, Acetylierung, Phosphorylierung, Amidierung, Palmitoylierung, Myristolylierung, Isoprenylierung, Lipidierung, Alkylierung, Derivatisierung, Einbringen von Schutz-/Blockierungsgruppen, proteolytische Spaltung oder Bindung an einen Antikörper oder an einen anderen zellulären Liganden entstehen. Derivate von Proteinen oder Polypeptiden können auch durch andere Verfahren wie beispielsweise durch chemische Spaltung mit Bromcyan, Trypsin, Chymotrypsin, Papain, V8-Protease, NaBH₂, Acetylierung, Formylierung, Oxidation, Reduktion oder durch metabolische Synthese in Gegenwart von Tunicamycin hergestellt werden.

Ferner erstreckt sich der Begriff "Derivat" auch auf alle funktionellen chemischen Äquivalente der Proteine oder Polypeptide.

Die vorstehend beschriebenen Derivate von Proteinen und Polypeptiden sind erfindungsgemäß von dem Begriff "Fusionsmolekül" umfasst, selbst wenn darauf nicht ausdrücklich verwiesen wird.

Die erfindungsgemäß beschriebenen pharmazeutischen Zusammensetzungen können therapeutisch für die Behandlung einer bereits bestehenden Erkrankung oder prophylaktisch als Vakzinen für die Immunisierung eingesetzt werden.

Der Begriff "Vakzine" betrifft erfindungsgemäß eine antigenische Zubereitung, die beispielsweise ein Protein, ein Peptid, eine Nukleinsäure oder ein Polysaccharid umfasst und die an einen Empfänger verabreicht wird, um dessen humorales und/oder zelluläres Immunsystem gegenüber einem oder mehreren Antigenen zu stimulieren, die in der Vakzinezubereitung vorliegen. Die Begriffe "Vakzinierung" oder "Immunisierung" betreffen den Vorgang einer Verabreichung einer Vakzine und der Stimulierung einer Immunreaktion gegenüber einem Antigen. Der Begriff "Immunreaktion" betrifft die Aktivitäten des Immunsystems, einschließlich Aktivierung und Proliferation von spezifischen cytotoxischen T-Zellen nach Kontakt mit einem Antigen.

Tiermodelle können für ein Testen einer immunisierenden Wirkung z.B. gegenüber Krebs bei Verwendung eines Tumor-assozüerten Antigens als Antigen eingesetzt werden. Dabei können beispielsweise menschliche Krebszellen in eine Maus für die Schaffung eines Tumors eingebracht werden und eine erfindungsgemäße Nukleinsäure, die für ein erfindungsgemäßes Fusionsmolekül, umfassend das Tumor-assoziiertes Antigen, kodiert, kann verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden ein oder mehrere Fusionsmoleküle mit einem oder mehreren Adjuvanzien für eine Induktion einer Immunreaktion oder eine Erhöhung einer immunreaktion verabreicht. Ein Adjuvans ist eine Substanz, die in ein Antigen eingebaut oder gemeinsam mit diesem verabreicht wird und die Immunreaktion verstärkt. Adjuvanzien können die Immunreaktion durch Bereitstellen eines Antigen-Reservoirs (extrazelluläre oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Freundsches Adjuvans, Vitamin E, Montanid, Alaun, CpG-Oligonukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Fusionsmoleküle in einem Gemisch mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Erfindungsgemäß umfasst eine Induktion einer Immunreaktion in einem Säuger im Allgemeinen die Verabreichung einer wirksamen Menge eines erfindungsgemäßen Fusionsmoleküls und/oder einer dafür kodierenden Nukleinsäure, insbesondere in Form eines Vektors. Vorzugsweise wird DNA oder RNA, die für ein erfindungsgemäßes Fusionsmolekül kodiert, an einen Säuger zusammen mit einer DNA-Sequenz verabreicht, die für einen T-Zell-co-stimulierenden Faktor kodiert, wie ein für B7-1 oder B7-2 kodierendes Gen.

Der Begriff "T-Zell-co-stimulierender Faktor" betrifft hier ein Molekül, insbesondere ein Peptid, das zur Bereitstellung eines co-stimulierenden Signals fähig ist und dadurch eine Immunreaktion verstärkt, insbesondere die Vermehrung von T-Zellen in Gegenwart eines oder mehrerer erfindungsgemäßer Fusionsmoleküle aktiviert. Eine derartige Aktivierung der T-Zell-Vermehrung kann durch allgemein bekannte Tests bestimmt werden.

Diese Faktoren umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD 152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Erfindungsgemäß vorgesehen ist eine Verabreichung von Nukleinsäuren, Polypeptiden oder Proteinen und/oder Zellen. Eine Verabreichung von DNA und RNA ist bevorzugt.

In den Experimenten konnte gezeigt werden, dass im Vergleich zu dem nicht-modifizierten Antigen erfindungsgemäß eine 100-fach geringere Dosis des Impfstoffs ausreicht, um äquivalente oder stärkere Immunantworten zu induzieren. Ein Problem bei der direkten Injektion von NukleinsäureImpfstoffen ist, dass die Dosis, die nötig ist, um Immunantworten zu induzieren, sehr hoch ist. Bei DNA-Impfstoffen ist die Ursache vermutlich hauptsächlich darin begründet, dass nur ein Bruchteil der Zellen injizierte DNA in den Kern aufnehmen. Bei RNA-Impfstoffen liegt das Problem vermutlich insbesondere darin, dass injizierte RNA sehr schnell durch RNAsen abgebaut wird.

Bei Verwendung der erfindungsgemäß modifizierten Impfstoffe ist zu erwarten, dass bei einer direkten Injektion von Nukleinsäuren, insbesondere RNA im Vergleich zu unmodifizierten Nukleinsäuren massiv höhere Immunantworten erhalten werden.

In einer bevorzugten Ausführungsform ist ein viraler Vektor für die Verabreichung einer Nukleinsäure, die für ein erfindungsgemäßes Fusionsmolekül kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um Nukleinsäuren in Zellen in vitro oder in vivo einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Vorzugsweise werden die Nukleinsäuren zusammen mit stabilisierenden Substanzen wie RNA-stabilisierenden Substanzen verabreicht.

In einer Ausführungsform erfolgt die Verabreichung von Nukleinsäuren durch ex vivo-Verfahren, d.h. durch Entfernung von Zellen aus einem Patienten, genetische Veränderung der Zellen, und Wiedereinbringung der veränderten Zellen in den Patienten. Dies umfasst im Allgemeinen das Einbringen einer funktionellen Kopie eines Gens in die Zellen eines Patienten in vitro und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren in vivo durch die Verwendung von Vektoren wie Viren und zielgesteuerten Liposomen.

Eine Verabreichung von Polypeptiden und Peptiden kann in an sich bekannter Weise erfolgen.

Der Begriff "Patient", "Individuum" oder "Lebewesen" bedeutet erfindungsgemäß Mensch, nicht menschlicher Primat oder ein anderes Tier, insbesondere Säugetier wie Kuh, Pferd, Schwein, Schaf, Ziege, Hund, Katze, Vögel wie Huhn oder Nagetier wie Maus und Ratte. In einer besonders bevorzugten Ausführungsform ist der Patient, das Individuum oder das Lebewesen ein Mensch.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan, intrakutan, transdermal, intralymphatisch, vorzugsweise durch Injektion in Lymphknoten, insbesondere Leistenlymphknoten, Lymphgefäße und/ oder in die Milz, erfolgen.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunrcaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA) erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nicht-toxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Akalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Vorzugsweise sind die Trägerstoffe sterile Flüssigkeiten wie Wasser oder Öle, einschließlich derjenigen, die sich von Erdöl, Tieren oder Pflanzen ableiten oder synthetischen Ursprungs sind, wie z.B. Erdnussöl, Sojabohnenöl, Mineralöl, Sesamöl, Sonnenblumenöl und dergleichen. Salzlösungen und wässrige Dextrose- und Glycerinlösungen können auch als wässrige Trägerstoffe verwendet werden.

Beispiele für Hilfs- und Trägerstoffe sind Acryl- und Methacrylderivate, Alginsäure, Sorbinsäurederivate wie α-Octadecyl-ω-hydroxypoly(oxyethylen)-5-sorbinsäure, Aminosäuren und deren Derivate, insbesondere Aminverbindungen wie Cholin, Lecithin und Phosphatidylcholin, Gummi arabicum, Arornastoffe, Ascorbinsäure, Carbonate wie beispielsweise Natrium-, Kalium-, Magnesium- und Calciumcarbonat und -hydrogencarbonat, Hydrogenphosphate und Phosphate von Natrium, Kalium, Calcium und Magnesium, Carmellosenatrium, Dimeticon, Farbstoffe, Geschmacksstoffe, Puffersubstanzen, Konservierungsmittel, Verdickungsmittel, Weichmacher, Gelatine, Glucosesirupe, Malz, hochdisperses Siliziumdioxid, Hydromellose, Benzoate, insbesondere Natrium- und Kaliumbenzoat, Macrogol, Magermilchpulver, Magnesiumoxid, Fettsäuren und deren Derivate und Salze wie Stearinsäure und Stearate, insbesondere Magnesium- und Calciumstearat, Fettsäureester sowie Mono-und Diglyceride von Speisefettsäuren, natürliche und künstliche Wachse wie Bienenwachs, gelbes Wachs und Montanglycolwachs, Chloride, insbesondere Natriumchlorid, Polyvidon, Polyethylenglykole, Polyvinylpyrrolidon, Povidon, Öle wie Rizinusöl, Sojaöl, Kokosnussöl, Palinkernöl, Zucker und Zuckerderivate, insbesondere Mono- und Disaccharide wie Glucose, Fructose, Mannose, Galactose, Lactose, Maltose, Xylose, Saccharose, Dextrose und Cellulose und deren Derivate, Schellack, Stärke und Stärkederivate, insbesondere Maisstärke, Talg, Talkum, Titandioxid, Weinsäure, Zuckeralkohole wie Glycerin, Mannit, Sorbit und Xylit und deren Derivate, Glykol, Ethanol und Gemische derselben.

Vorzugsweise können die pharmazeutischen Zusammensetzungen zusätzlich auch Benetzungsmittel, Emulgatoren und/oder pH-puffernde Mittel enthalten.

In einer weiteren Ausführungsform können die pharmazeutischen Zusammensetzungen einen Resorptionsverstärker enthalten. Diese Resorptionsverstärker können, falls gewünscht, eine äquimolare Menge des Trägerstoffs in der Zusammensetzung ersetzen. Beispiele für solche Resorptionsverstärker umfassen in nicht begrenzender Weise Eucalyptol, N,N-Diethyl-m-toluamid, Polyoxyalkylenalkohole (wie Propylenglykol und Polyethylenglykol), N-Methyl-2-pyrrolidon, Isopropylmyristat, Dimethylformamid (DMF), Dimethylsulfoxid (DMSO), Dimethylacetamid (DMA), Harnstoff, Diethanolamin, Triethanolamin und dergleichen (siehe z.B. Percutaneous Penetration Enhancers, Hrsg. Smith et al. (CRC Press, 1995)). Die Menge an Resorptionsverstärker in der Zusammensetzung kann von den gewünschten zu erreichenden Wirkungen abhängen.

Ein Protease-Inhibitor kann in die erfindungsgemäße Zusammensetzung eingebaut werden, um einen Abbau eines Peptid- oder Proteinwirkstoffs zu vermeiden und dadurch die Bioverfügbarkeit zu erhöhen. Beispiele für Protease-Inhibitoren umfassen in nicht-begrenzender Weise Aprotinin, Leupepsin, Pepstatin, a2-Makroglobulin und Trypsin-Inhibitor. Diese Inhibitoren können alleine oder in Kombination verwendet werden.

Die erfindungsgemäßen pharmazeutischen Zusammensetzungen können mit einer oder mehreren Beschichtungen versehen sein. Vorzugsweise sind die festen oralen Darreichungsformen mit einer magensaftresistenten Beschichtung versehen oder liegen in Form einer magensaftresistenten, gehärteten Weichgelatinekapsel vor.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringes-lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Beispiele und Figuren ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die nicht über den Rahmen der Erfindung und den Umfang der anhängenden Ansprüche hinausgehen.

### Kurze Beschreibung der Zeichnungen:

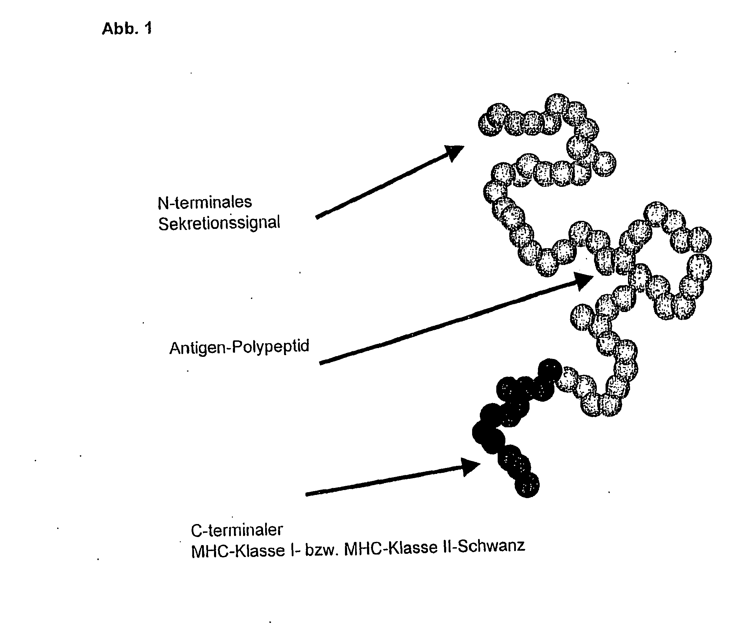

**Abbildung 1****: Schematische Darstellung eines erfindungsgemäßen Fusionsproteins.** Das Fusionsprotein besteht aus einem N-terminal gelegenen Sekretionssignal, einer C-terminal lokalisierten Transmembran-und cytoplasmatischen Domäne eines Histokompatibilitätsantigens und einer integrierten kompletten oder partiellen Sequenz eines Antigens.

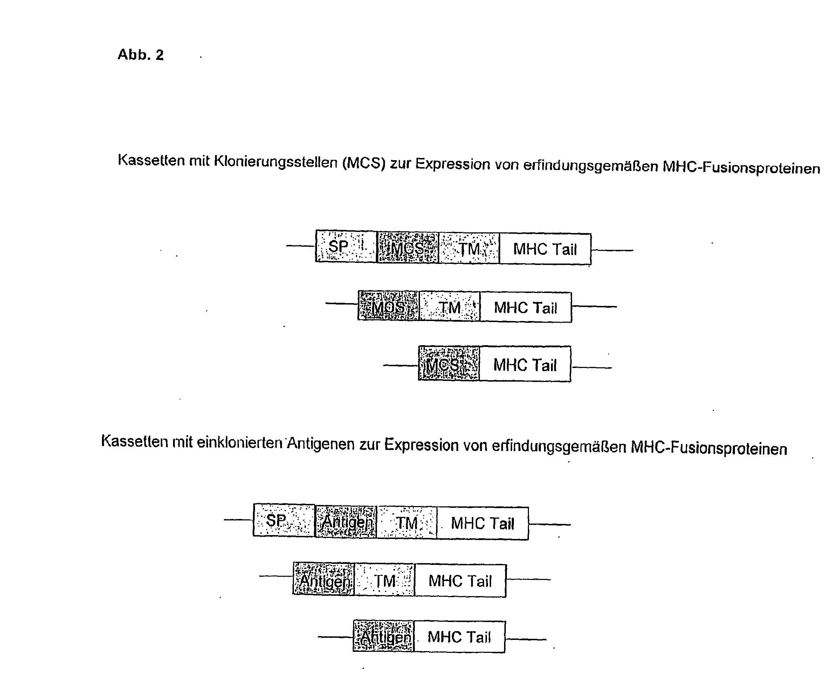

**Abbildung 2****: Schematische Darstellung der Kassetten für die Expression von Fusionsproteinen.** SP: Signalpeptid; MCS: multiple Klonierungsstelle; TM: Transmembrandomäne; MHC Tail: cytoplasmatischer Schwanz eines MHC-Moleküls; Antigen: Sequenz kodierend für ein Antigen, gegen das Immunantworten induziert werden sollen

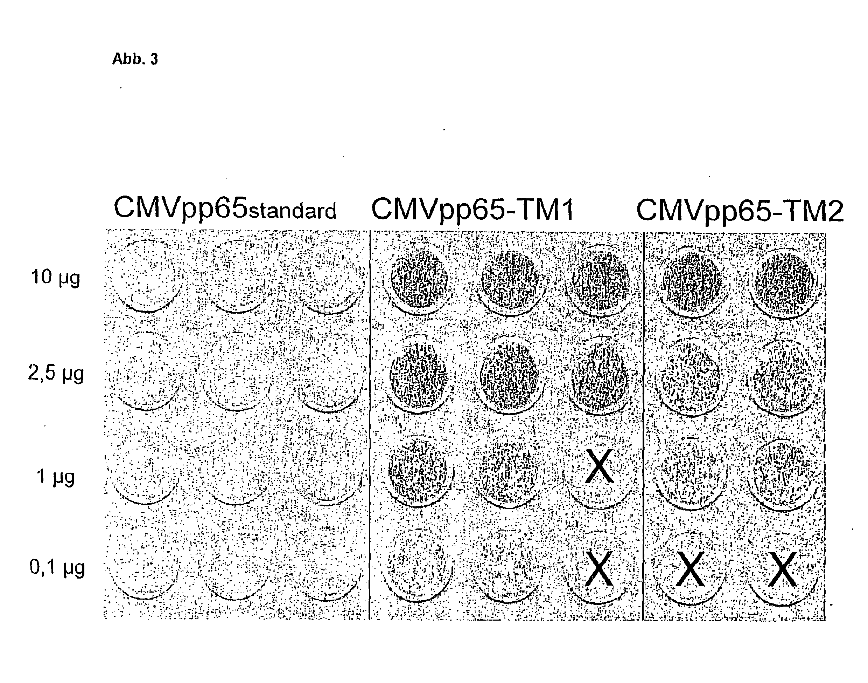

**Abbildung 3****: Testen der Auswirkung unterschiedlicher RNA-Dosen auf die Frequenz antigenspezifischer CD4+-T-Lymphozyten.**
   1 x 10⁶ aufgereinigte CD4+-Lymphozyten wurden über 1 Woche mit 2 x 10⁵ DC kokultiviert, welche mit RNA in den angegebenen Mengen (0,1-10 µg RNA) per Elektroporation transfiziert worden waren. Am Tag 7 nach Stimulation wurde ein ELISPOT unter Standardbedingungen zum Nachweis Interferon-gamma-sezernierender T-Lymphozyten durchgeführt. Als Antigen-präsentierende Zellen wurden DC desselben Spenders, die mit überlappenden pp65-Peptiden (1,75 µg/ml) oder einem irrelevanten Kontrollpeptid beladen worden waren, verwendet. Für den Test wurden 3 x 10⁴ Effektoren mit 2 x 10⁴ DC für 16 h koinkubiert. Nach Standardentwicklung wurde die Anzahl der IFN-gamma-sezernierenden T-Lymphozyten mittels einer Software-basierten Videoauswertung bestimmt. Im Vergleich zu der CMVpp65standard-RNA zeigt sich eine massive Expansion von CD4+-Lymphozyten sowohl durch das CMVpp65-TM1- als auch durch das CMVpp65-TM2-Konstrukt.

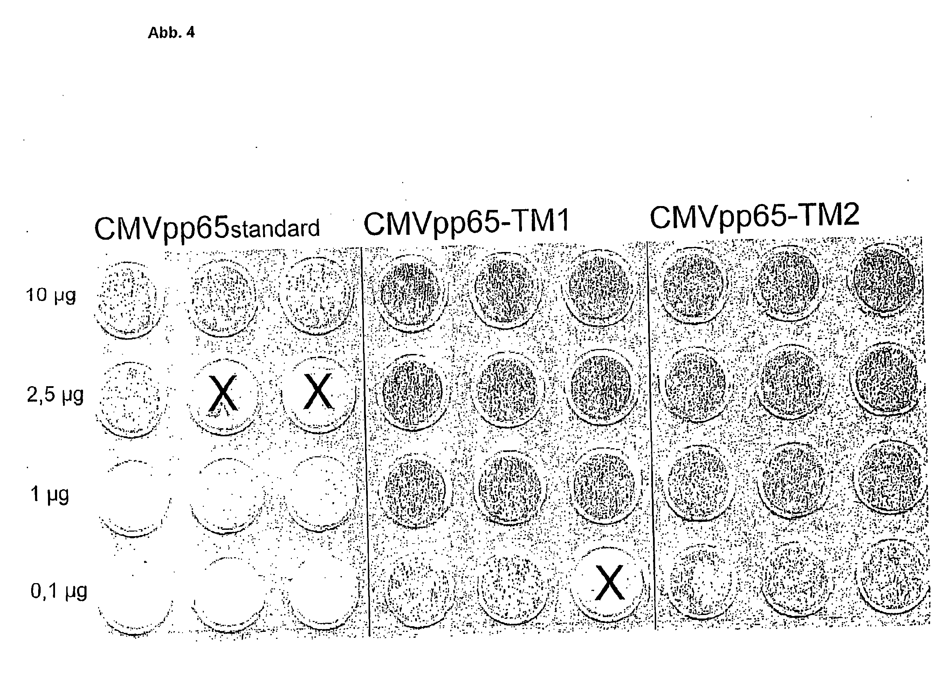

**Abbildung 4****: Testen der Auswirkung unterschiedlicher RNA-Dosen auf die Frequenz Interferon-gamma-sezernierender CD8+-T-Lymphozyten**. 1 x 10⁶ aufgereinigte CD8+-Lymphozyten wurden über 1 Woche mit 2 x 10⁵ DC kokultiviert, welche mit RNA in den angegebenen Mengen (0,1-10 µg RNA) per Elektroporation transfiziert worden waren. Am Tag 7 wurde ein Standard ELISPOT zum Nachweis IFN-gamma-sezemierender T-Lymphozyten gegen DC desselben Spenders durchgeführt, welche mit überlappenden pp65-Peptiden (1,75 µg/ml) oder einem irrelevanten Kontrollpeptid beladen worden waren. Es wurden 3 x 10⁴ Effektoren mit 2 x 10⁴ DC für 16 h koinkubiert. Nach Standardentwicklung wurde die Anzahl der IFN-gamma-sezernierenden T-Lymphozyten mittels einer Software-basierten Videoauswertung bestimmt. Es zeigte sich eine massive Expansion von CD8+-Lymphozyten durch das CMVpp65-TM1- und das CMVpp65-TM2-Konstrukt. Selbst bei Verwendung von 100x geringeren Dosen (0,1 µg RNA) liegt die Frequenz der pp65-spezifischen CD8+-Lymphozyten noch über dem Hintergrund nach Stimulation durch mit NYESO-RNA transfizierten DC (Daten nicht gezeigt). Die Stimulation durch das CMVpp65standard-Konstrukt zeigte erst ab 2,5 µg eine Expansion von pp65-spezifischen Lymphozyten über das Niveau des Hintergrunds hinaus.

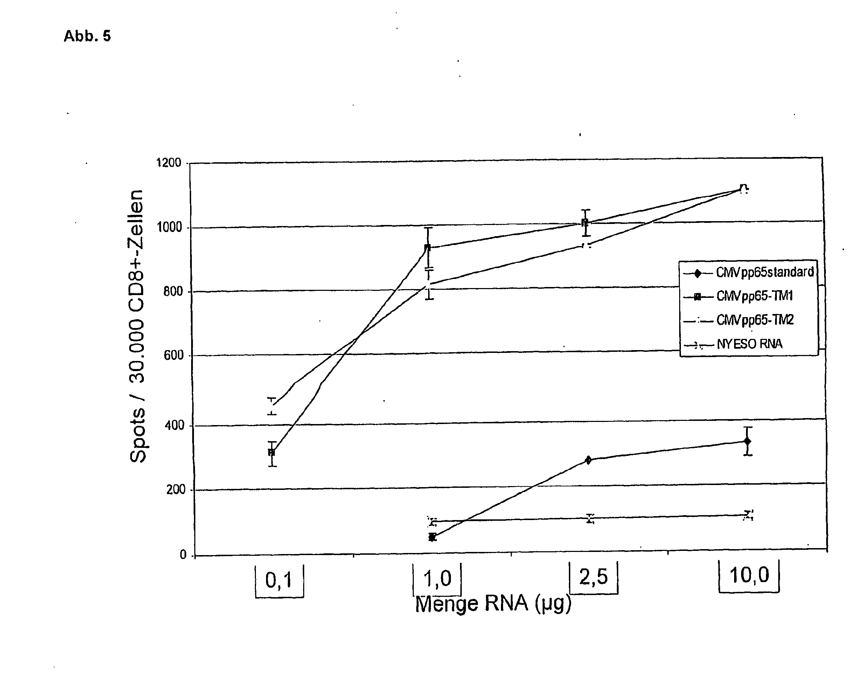

**Abbildung 5****: Dosis/Wirkungs-Profil für die Expansionskapazität verschiedener Immunogene auf Antigen-spezifische Lymphozyten.** Die erfindungsgemäß modifizierten Immunogene weisen eine deutlich gesteigerte Potenz (>100x) und eine höhere maximale Wirkung auf.

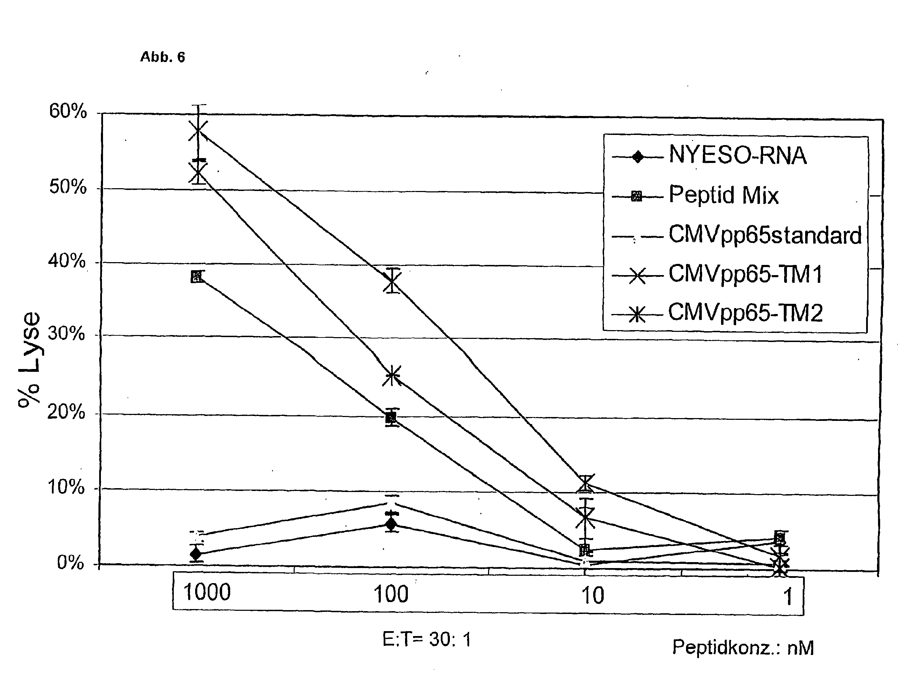

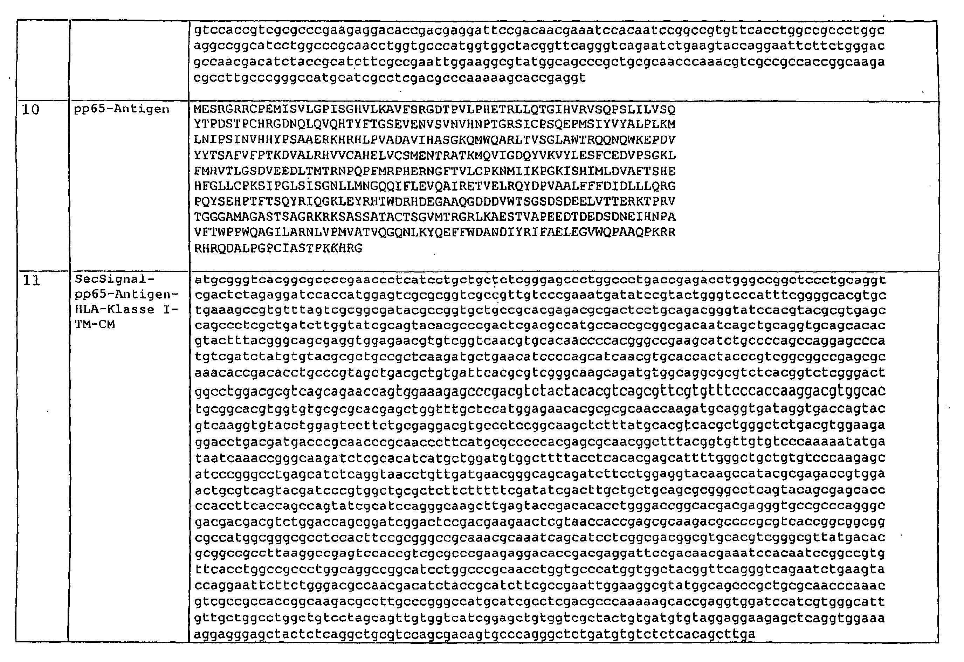

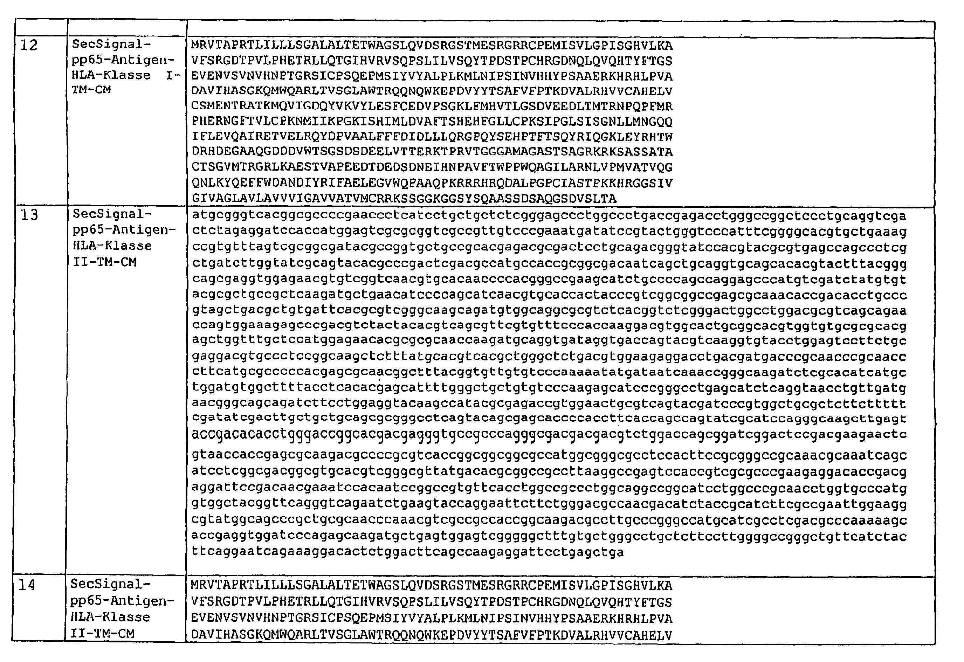

**Abbildung 6****: Vergleichender Test der Auswirkung von erfindungsgemäß modifizierten Immunogenen und Standardimmunogenen auf die Generierung von cytotoxischen Immunantworten.** 1 x 10⁶ aufgereinigte CD8+-Lymphozyten wurden über 1 Woche mit 2 x 10⁵ DC kokultiviert, welche mit 10µg RNA per Elektroporation transfiziert worden waren. Am Tag 7 wurde ein Standard Cytochrom-Cytotoxizitästest gegen DC desselben Spenders durchgeführt, welche mit unterschiedlichen Konzentrationen überlappender pp65-Peptide oder einem irrelevanten Kontrollpeptid beladen worden waren. Es wurden 15 x 10⁴ Effektoren mit 0,5 x 10⁴ DC für 4 h koinkubiert Nach Messung des Überstandes im Counter wurde die spezifische Lyse berechnet, gemäß der Formel: Es zeigte sich eine starke Lyse durch CD8+-Lymphozyten, welche mit CMVpp65-TM1- und CMVpp65-TM2-Konstrukten stimuliert worden waren, die bis zu einer Konzentration von 10 nM des pp65-Peptid-Gemisches über dem Wert für das Kontrollpeptid lag (Daten nicht gezeigt). Durch das pp65-Peptid-Gemisch wurden ebenfalls CD8+-Lymphozyten expandiert, die eine deutliche spezifische Lyse zeigten, aber nicht das Niveau von CMVpp65-TM 1 und -TM2 erreichten. Durch das CMVpp65standard-Konstrukt konnte nur eine schwache Stimulation pp65-spezifischer cytotoxischer T-Zellen erreicht werden.
**Abbildung 7: In den Beispielen verwendete Sequenzen HLA-Klasse I-TM-CM: Transmembran-cytoplasmatische Region eines HLA-Klasse I-Moleküls; HLA-Klasse II-TM-CM: Transmembran-cytoplasmatische Region eines HLA-Klasse II-Moleküls**

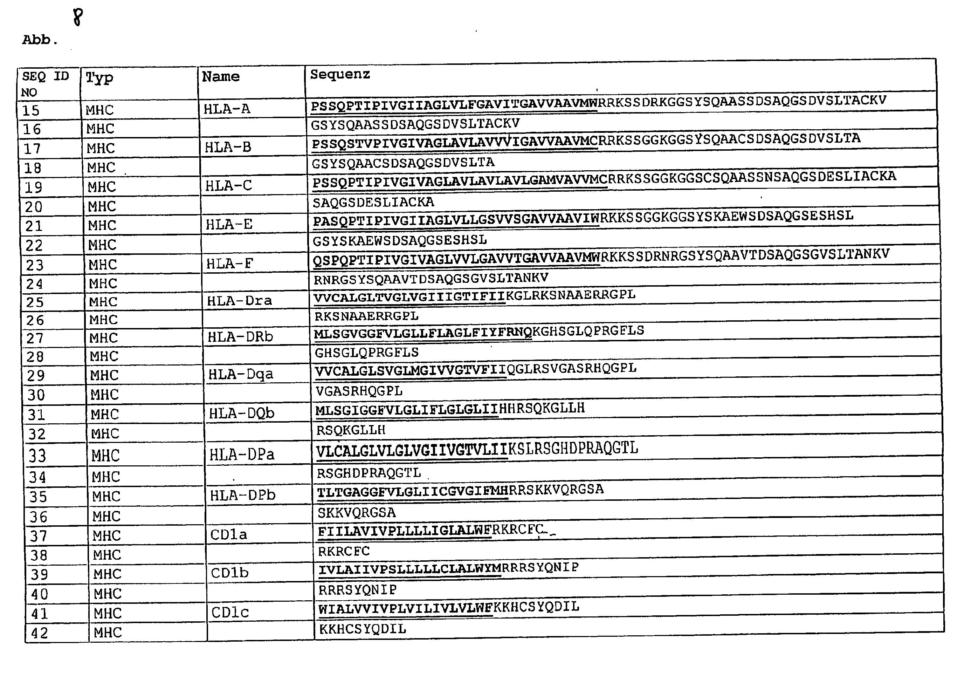

**Abbildung 8****: Sequenzen von Transmembran-cytoplasmatischen Regionen bzw. cytoplasmatischen Regionen von MHC-Molekülen.** Die Sequenzen zeigen die Transmembran-cytoplasmatische Region bzw. nur die cytoplasmatische Region verschiedener HLA Moleküle. Die Transmembranregion ist unterstrichen und fett

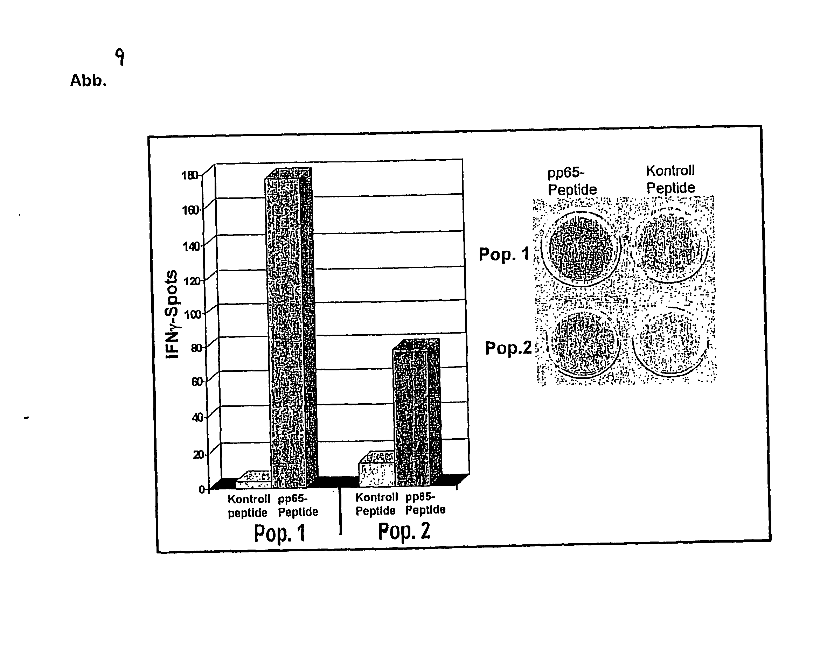

**Abbildung 9****: Stimulation naiver CD8⁺-T-Lymphozyten durch erfindungsgemäße Fusionskonstrukte.** In Mikrotiterplatten wurden pro Well 1x10⁵ CD8⁺-Lymphozyten gegen 2 x 10⁴ DC, welche mit 20 µg CMVpp65-TM1 oder Kontroll-RNA transfiziert waren, stimuliert. Das Medium wurde mit IL-6 (1000 U/ml) und IL-12 (10 ng/ml) supplementiert. Am Tag +7 und +14 wurde mit aufgetauten transfizierten DC (2 x 10⁴ / Well) restimuliert, wobei das Medium IL-2 (10 U/ml) und IL-7 (5 ng/ml) enthielt. Am Tag +21 wurden alle Populationen im ELISpot gegen Kontroll-Peptide (1,75µg/ml) und gegen pp65-überlappende Peptide (1,75µg/ml) getestet. Zwei der gegen CMVpp65-TM1 stimulierten Populationen (Pop.1, Pop.2) zeigten eine deutliche pp65-Reaktivität.

### Beispiele:

### Beispiel 1: Herstellung der modifizierten Vakzine

Für die Herstellung der modifizierten Vakzine wurde zunächst in einem Expressionsvektor, welcher die Transkription von RNA erlaubt, eine Kassette hergestellt, die die Expression von Fusionsgenen erlaubt. Hierfür wurde zunächst die Nukleinsäure, die für ein Signalpeptid eines HLA-Moleküls kodiert, aus menschlichen Lymphozyten amplifiziert und das Fragment als cDNA in einen Vektor kloniert (SEQ ID NO: 1 und 2). Die Klonierung wurde so durchgeführt, dass hinter der cDNA des Signalpeptids verschiedene Restriktionsenzym-Schnittstellen lagen und sich weitere Fragmente "inframe" in die Expressionskassette klonieren lassen. Als Vektoren wurden Plasmide ausgewählt, die über einen 5'-gelegenen RNA-Polymerase-Promoter T3, T7 bzw. SP6 eine in vitro-Expression von RNA erlauben. Als nächstes Fragment wurde eine cDNA in diesen Vektor kloniert, welche eine Transmembrandomäne und die cytoplasmatische Domäne eines HLA-Klasse I (SEQ ID NO: 3 und 4) bzw. eines Klasse II (SEQ ID NO: 5 und 6) Moleküls, einschließlich Stop-Codon kodiert. Die Klonierung wurde so durchgeführt, dass das entstehende Plasmid zwischen den beiden Fragmenten noch Restriktionsenzym-Schnittstellen zur Klonierung von Antigenen (SEQ ID NO: 7 und 8 und Abbildung 1) aufweist. In diese Expressionskassetten wurde als Modellantigen die für das humane Cytomegalovirus Phosphoprotein 65 (pp65) kodierende Sequenz (SEQ ID NO: 9 und 10) so einkloniert, dass ein durchgehender ORF aus HLA-Signalsequenz, pp65 und HLA-Transmembran- und cytoplasmatischer Domäne (SEQ ID NO: 11 und 12) entstand. Für Kontrollexperimente wurde ein Vektor hergestellt, der die pp65-Sequenz mit einem STOP-Codon in demselben Ausgangsvektor ohne die besagten Fragmente enthielt. Für die weitergehenden Experimente wurden folgende Nukleinsäuren genutzt:
**CMVpp65standard:** unmodifizierte CMVpp65-Sequenz, Standardimmunogen
**CMVpp65-TM1:** Fusionsnukleinsäure aus folgenden Fragmenten: HLA-Klasse I-Sekretionssignal, pp65-ORF und HLA-Klasse I-Transmembran- und cytoplasmatische Domäne (modifiziertes Immunogen).
**CMVpp65-TM2:** Fusionsnukleinsäure aus folgenden Fragmenten: HLA-Klasse I-Sekretionssignal, pp65-ORF und HLA-Klasse II-Transmembran- und cytoplasmatische Domäne (modifiziertes Immunogen).

### Beispiel 2: Testen der modifizierten Vakzine

Die drei Nukleinsäuren (CMVpp65standard, CMVpp65TM1, CMVpp65TM2) wurden als Immunogen in Stimulationsversuchen mit autologen DC's antigenpositiver Spender eingesetzt. Um CD4- und CD8-Iminunantworten getrennt zu testen, wurden aufgereinigte CD4+- und CD8+-Lymphozyten genutzt. Als Read-Out wurde der Enzyme-linked-Immuno-Spot-Assay (ELISPOT) eingesetzt, der als Standardtest zur Quantifizierung IFN-λ-sezernierender T-Zellen, anerkannt ist. Zum Testen der Effektorfunktion von CD8+-T-Lymphozyten wurde ein Standard-Chrom-Freisetzungstest benutzt. Autologe Monozyten oder DC's wurden mit pp65-RNA, CMVpp65-TM1- und CMVpp65-TM2-Immunogenen transfiziert. Als Maximal-Stimulationskontrolle wurden DC's mit überlappenden Peptiden für pp65 sowie mit Kontrollpeptid beladen. Die so behandelten DC's wurden über Nacht oder für 7 Tage mit CD4+- oder CD-8+-Lymphozyten koinkubiert. Das Read-Out erfolgte gegen autologe Monozyten oder DC's, die mit pp65-überlappenden Peptiden oder einem CMV-Fibroblastenlysat gepulst worden waren. Bei der Untersuchung von CD4+-Immunantworten zeigte sich überraschenderweise, dass beide modifizierten Immunogene (CMVpp65-TM1 und CMVpp65-TM2) nicht nur eine gegenüber dem CMVpp65standard-Immunogen verstärkte Immunantwort auslösten, sondern auch eine maximal starke antigenspezifische IFN-gamma-Sekretion bei CD4+-Lymphozyten induzierten (Abbildung 3). Der Prozentsatz der antigenspezifischen CD4+-Zellen nach Stimulation durch die modifizierten pp65-Konstrukte war dabei gleich oder sogar höher als nach Stimulation mit pp65-überlappenden Peptiden. Wie erwartet, zeigte das CMVpp65standard-Immunogen keine relevante Stimulation von CD4+-Lymphozyten.

Ein noch überraschenderes Ergebnis ergab sich bei der Untersuchung von CD8-Immunantworten nach Stimulation mit den Immunogenen. Es ließ sich zeigen, dass die Verwendung der modifizierten Expressionskassetten zur Stimulation von CD8+-Lymphozyten ebenfalls zu einem Anteil spezifisch IFN-λ-sezernierender Zellen führte, welcher dem nach Stimulation mit pp65-überlappenden Peptiden vergleichbar ist. Überraschenderweise waren auch hierbei die modifizierten RNA-Konstrukte den unveränderten CMVpp65standard-Immunogenen weit überlegen (Abbildungen 4 und 5). Die Ergebnisse im Zytotoxizitätsassay zeigten, dass beide Modifikationen zur einer bisher noch nicht beschriebenen drastischen Erhöhung der Cytotoxizität im Vergleich zu CMVpp65standard-RNA führten (Abbildung 6). Auch hierbei zeigte sich überraschenderweise eine Überlegenheit der modifizierten Immunogene gegenüber den überlappenden pp65-Peptiden.

### Beispiel 3: Stimulation naiver CD8⁺-T-Lymphozyten durch HLA-Fusionsantigene

Um die Möglichkeit des Priming und der nachfolgenden Expansion von naiven CD8⁺-Lymphozyten durch die erfindungsgemäßen Fusionskonstrukte zu testen, wurden dendritische Zellen eines CMV-negativen Spenders mit RNA des unmodifizierten CMVpp65, oder mit CMVpp65-TM1-RNA bzw. mit einer Kontroll-RNA (NY-Eso-1) transfiziert. Die transfizierten dendritischen Zellen wurden zur Stimulation von autologen CD8⁺-Lymphozyten eingesetzt. Es wurden 2 Restimulationen in wöchentlichem Abstand mit eingefrorenen transfizierten dendritischen Zellen durchgeführt. Zum Read-Out wurden am Tag +21 nach der ersten Stimulation alle Zell-Populationen in einem IFNγ-ELISpot Assay gegen autologe dendritische Zellen getestet, welche entweder mit pp65-überlappenden Peptiden oder zur Kontrolle mit irrelevanten überlappenden Peptiden beladen waren. Hierbei wurde festgestellt, dass durch die Stimulation mit CMVpp65-TM1-RNA in zwei Fällen pp65-reaktive CD8+-T-Lymphozyten-Populationen generiert wurden (Abbildung 9). Stimulationen mit der dendritischen Zellen, die mit der unmodifizierten CMVpp65 RNA bzw. mit Kontroll-RNA transfizierte wurden, wiesen dagegen keine signifikante pp65-Reaktivität auf.

### Beispiel 4: Nutzung von HLA-Fusionsantigenen zur Stimulation von tumorzellreaktiven T-Lymphozyten

Um CD8⁺- und CD4⁺-T-Lymphozyten gegen definierte Tumorantigene expandieren zu können, wurden folgende Antigensequenzen als Inserts in erfindungsgemäße Fusionskonstrukte kloniert: das Tumorantigen TPTE (Koslowski et al., 2004, PMID 15342378), das Tumorantigen PRAME (Ikeda et al., 1997, PMID 9047241) in der Variante 1 (SEQ ID NO: 43), das Tumorantigen WT1 als Variante C (SEQ ID NO: 44) sowie das Tumorantigen p53 (SEQ ID NO: 45). Für die funktionelle Validierung wurden humane dendritische Zellen eines HLA* A O2O1-positiven Spenders entweder mit WT1-HLA-TM1-RNA, mit unmodifizierter WT1-RNA oder irrelevanter Kontroll-RNA transfiziert und als Targetzellen benutzt. Nach Koinkubation mit WT1-reaktiven CD8⁺-T-Zellklonen für 8 oder 16 Stunden wurde IFNγ im Überstand quantifiziert. Es zeigte sich, dass nach Koinkubation mit WT1-HLA-TM1-transfizierten dendritischen Zellen die Sekretion um einen Faktor 6-9 höher lag im Vergleich zur Koinkubation nach Transfektion mit unmodifiziertem WT1.

In einer Serie von Experimenten wurden zusammenfassend mehrfach bestätigt folgende Resultate erzielt:
- Die modifizierten Immunogene führen zu einer deutlich verstärkten Stimulation und Expansion von Antigen-spezifischen CD4+-Lymphozyten (gesteigerte Vermehrung von CD4+-Lymphozyten)
- Die modifizierten Immunogene führen zu einer deutlich verstärkten Stimulation und Expansion von Antigen-spezifischen CD8+-Lymphozyten (gesteigerte Vermehrung von CD8+-Lymphozyten)
- Die modifizierten Immunogene führen zu einer deutlich verstärkten Zytokinausschüttung von Antigen-spezifischen CD4+-Lymphozyten und CD8+-Lymphozyten (gesteigerte Zytokinausschüttung=gesteigerte Aktivierung)
- Die modifizierten Immunogene führen zu einer deutlich verstärkten cytotoxischen Reaktivität von Antigen-spezifischen CD8+-Lymphozyten (gesteigerter zytotoxische Effekt)
- Die modifizierten Immunogene sind 100x potenter bezüglich der Expansion von Antigen-spezifischen CD8+-Lymphozyten
- Die modifizierten Immunogene haben selbst bei 100x niedrigerer Dosis einen stärkeren Effekt auf die Expansion von Antigen-spezifischen CD4+-Lymphozyten als Standard-Immunogene

Zusammenfassend lässt sich also sagen, dass die erfindungsgemäßen Modifikationen eines Antigens in einer über 100-fach gesteigerten Potenz (Linksverschiebung der Dosis-Wirkungskurve) und einer drastisch erhöhten biologischen Wirksamkeit resultieren. Im Vergleich mit den bisher üblichen unmodifizierten Antigensequenzen lässt sich ein Immunogen generieren, das als Vakzine eine quantitativ und qualitativ höhere Wirksamkeit besitzt.

Ein wichtiges Resultat der Erfindung ist, dass gleichzeitig antigenspezifische CD4+- und CD8+-Lymphozyten optimal stimuliert und expandiert werden. Für die Wirksamkeit besonders von therapeutischen Vakzinen ist eine Stimulation der CD8+- und CD4+-Lymphozyten von entscheidender Bedeutung.

## Patentansprüche

1. Fusionsmolekül, das ein Antigen, eine Transmembranregion und die cytoplasmatische Region einer Kette eines MHC-Moleküls umfasst, wobei das Fusionsmolekül keines der folgenden umfasst: (i) α1-, α2-, und α3-Domänen der α-Kette eines MHC-Klasse 1-Moleküls, (ii) β2-Mikroglobulin eines MHC-Klasse 1-Moleküls, (iii) α1- und α2-Domänen der α-Kette eines MHC-Klasse II-Moleküls, und (iv) β1- und β2-Domänen der β-Kette eines MHC-Klasse II-Moleküls.

2. Fusionsmolekül nach Anspruch 1, wobei die Transmembranregion eine Transmembranregion eines MHC-Moleküls ist.

3. Fusionsmolekül nach Anspruch 1 oder 2, wobei die Transmembranregion und cytoplasmatische Region eine Sequenz umfassen, die der Transmembranregion in Verbindung mit der cytoplasmatischen Region eines MHC-Moleküls entspricht.

4. Fusionsmolekül nach einem der Ansprüche 1 bis 3, wobei das Fusionsmolekül zusätzlich eine Leitsequenz umfasst, die in der Lage ist, die Translokation eines Proteins oder Peptids durch eine Membran zu steuern.

5. Fusionsmolekül nach Anspruch 4, wobei die Leitsequenz eine Leitsequenz eines MHC-Moleküls ist.

6. Fusionsmolekül nach Anspruch 4 oder 5, wobei das Fusionsmolekül folgende Anordnung aufweist: N-Terminus-Leitsequenz / Antigen /Transmembranregion / cytoplasmatische Region-C-Terminus, wobei die einzelnen Regionen gegebenenfalls durch Linkersequenzen voneinander getrennt sein können.

7. Nukleinsäure, die für ein Fusionsmolekül nach einem der Ansprüche 1 bis 6 kodiert.

8. Wirtszelle, die eine Nukleinsäure nach Anspruch 7 umfasst.

9. Pharmazeutische Zusammensetzung, die ein oder mehrere Fusionsmoleküle nach einem der Ansprüche 1 bis 6 und/oder eine oder mehrere Nukleinsäuren nach Anspruch 7 und/oder eine oder mehrere Wirtszellen nach Anspruch 8 umfasst.

10. Pharmazeutische Zusammensetzung nach Anspruch 9 in Form einer Vakzine.

11. In vitro-Verfahren zur Erhöhung der Menge an MHC/Peptid-Komplexen in einer Zelle, wobei das Verfahren die Bereitstellung eines oder mehrerer Fusionsmoleküle nach einem der Ansprüche 1 bis 6 und/oder einer oder mehrerer Nukleinsäuren nach Anspruch 7 für die Zelle umfasst.

12. Verwendung eines oder mehrerer Fusionsmoleküle nach einem der Ansprüche 1 bis 6 und/oder einer oder mehrerer Nukleinsäuren nach Anspruch 7 zur Herstellung einer pharmazeutischen Zusammensetzung zur Erhöhung der Menge an MHC/Peptid-Komplexen in einer Zelle.

13. In vitro-Verfahren zur Steigerung der Präsentation von Zelloberflächenmolekülen auf Zellen, die in der Lage sind, Antigene zu präsentieren, insbesondere B-Zellen und Makrophagen, wobei das Verfahren die Bereitstellung eines oder mehrerer Fusionsmoleküle nach einem der Ansprüche 1 bis 6 und/oder einer oder mehrerer Nukleinsäuren nach Anspruch 7 für die Zellen umfasst.

14. Verwendung eines oder mehrerer Fusionsmoleküle nach einem der Ansprüche 1 bis 6 und/oder einer oder mehrerer Nukleinsäuren nach Anspruch 7 zur Herstellung einer pharmazeutischen Zusammensetzung zur Steigerung der Präsentation von Zelloberflächenmolekülen auf Zellen, die in der Lage sind, Antigene zu präsentieren, insbesondere B-Zellen und Makrophagen.

15. Verfahren nach Anspruch 11 oder 13 oder Verwendung nach Anspruch 12 oder 14, wobei die Erhöhung der Menge an MHC/Peptid-Komplexen oder die Steigerung der Präsentation von Zelloberflächenmolekülen ihrerseits die primäre Aktivierung von T-Zellen, insbesondere von CD4⁺- und CD8⁺-Lymphozyten, die gegenüber dem Antigen reagieren, verstärkt.

16. Verwendung eines oder mehrerer Fusionsmoleküle nach einem der Ansprüche 1 bis 6 und/oder einer oder mehrerer Nukleinsäuren nach Anspruch 7 und/oder einer oder mehreren Wirtszellen nach Anspruch 8 zur Herstellung einer pharmazeutischen Zusammensetzung zum Auslösen einer Immunreaktion bei einem Lebewesen.

17. In vitro-Verfahren zur Stimulierung oder Aktivierung von T-Zellen, insbesondere CD4⁺- und CD8⁺-Lymphozyten, wobei das Verfahren die Bereitstellung für die T-Zellen eines oder mehrerer Fusionsmoleküle nach einem der Ansprüche 1 bis 6 und/oder einer oder mehrerer Nukleinsäuren nach Anspruch 7 und/oder einer oder mehrerer Wirtszellen nach Anspruch 8 umfasst.

18. Verwendung eines oder mehrerer Fusionsmoleküle nach einem der Ansprüche 1 bis 6 und/oder einer oder mehrerer Nukleinsäuren nach Anspruch 7 und/oder einer oder mehrerer Wirtszellen nach Anspruch 8 zur Herstellung einer pharmazeutischen Zusammensetzung zur Stimulierung oder Aktivierung von T-Zellen, insbesondere CD4⁺- und CD8⁺-Lymphozyten, in einem Lebewesen.

19. Verwendung eines oder mehrerer Fusionsmoleküle nach einem der Ansprüche 1 bis 6 und/oder einer oder mehrerer Nukleinsäuren nach Anspruch 7 und/oder einer oder mehrerer Wirtszellen nach Anspruch 8 zur Herstellung einer pharmazeutischen Zusammensetzung zur Behandlung, Vakzinierung oder Immunisierung eines Lebewesens.

## Claims

1. A fusion molecule which comprises an antigen, a transmembrane region and the cytoplasmic region of a chain of an MHC molecule, wherein the fusion molecule does not comprise any of the following: (i) α1, α2 and α3 domains of the α chain of an MHC class I molecule, (ii) β2-microglobulin of an MHC class I molecule, (iii) α1 and α2 domains of the α chain of an MHC class II molecule and (iv) β1 and β2 domains of the β chain of an MHC class II molecule.

2. The fusion molecule of claim 1, wherein the transmembrane region is a transmembrane region of an MHC molecule.

3. The fusion molecule of claim 1 or 2, wherein the transmembrane region and cytoplasmic region comprise a sequence which corresponds to the transmembrane region connected to the cytoplasmic region of an MHC molecule.

4. The fusion molecule of any one of claims 1 to 3, wherein the fusion molecule additionally comprises a leader sequence which is able to control translocation of a protein or peptide through a membrane.

5. The fusion molecule of claim 4, wherein the leader sequence is a leader sequence of an MHC molecule.

6. The fusion molecule of claim 4 or 5, wherein the fusion molecule has the following arrangement: N terminus - leader sequence / antigen / transmembrane region / cytoplasmic region - C terminus, wherein the individual regions may optionally be separated from one another by linker sequences.

7. A nucleic acid which codes for a fusion molecule of any one of claims 1 to 6.

8. A host cell which comprises a nucleic acid of claim 7.

9. A pharmaceutical composition which comprises one or more fusion molecules of any one of claims 1 to 6 and/or one or more nucleic acids of claim 7 and/or one or more host cells of claim 8.

10. The pharmaceutical composition of claim 9 in the form of a vaccine.

11. An in vitro method for increasing the amount of MHC/peptide complexes in a cell, wherein the method comprises the provision of one or more fusion molecules of any one of claims 1 to 6 and/or of one or more nucleic acids of claim 7 for the cell.

12. Use of one or more fusion molecules of any one of claims 1 to 6 and/or of one or more nucleic acids of claim 7 for the preparation of a pharmaceutical composition for increasing the amount of MHC/peptide complexes in a cell.

13. An in vitro method for increasing the presentation of cell surface molecules on cells which are able to present antigens, in particular B cells and macrophages, wherein the method comprises the provision of one or more fusion molecules of any one of claims 1 to 6 and/or of one or more nucleic acids of claim 7 for the cells.

14. Use of one or more fusion molecules of any one of claims 1 to 6 and/or of one or more nucleic acids of claim 7 for the preparation of a pharmaceutical composition for increasing the presentation of cell surface molecules on cells which are able to present antigens, in particular B cells and macrophages.

15. The method of claim 11 or 13 or the use of claim 12 or 14, wherein the increase in the amount of MHC/peptide complexes or the increase in the presentation of cell surface molecules in turn enhances the primary activation of T cells, in particular of CD4⁺ and CD8⁺ lymphocytes, which respond to the antigen.

16. Use of one or more fusion molecules of any one of claims 1 to 6 and/or of one or more nucleic acids of claim 7 and/or of one or more host cells of claim 8 for the preparation of a pharmaceutical composition for inducing an immune response in a living creature.

17. An in vitro method for stimulating or activating T cells, in particular CD4⁺ and CD8⁺ lymphocytes, wherein the method comprises the provision for the T cells of one or more fusion molecules of any one of claims 1 to 6 and/or of one or more nucleic acids of claim 7 and/or of one or more host cells of claim 8.

18. Use of one or more fusion molecules of any one of claims 1 to 6 and/or of one or more nucleic acids of claim 7 and/or of one or more host cells of claim 8 for the preparation of a pharmaceutical composition for stimulating or activating T cells, in particular CD4⁺ and CD8⁺ lymphocytes, in a living creature.

19. Use of one or more fusion molecules of any one of claims 1 to 6 and/or of one or more nucleic acids of claim 7 and/or of one or more host cells of claim 8 for the preparation of a pharmaceutical composition for the treatment, vaccination or immunization of a living creature.

## Revendications

1. Molécule de fusion, qui comprend un antigène, une région transmembranaire et la région cytoplasmique d'une chaîne d'une molécule de CMH, tandis que la molécule de fusion ne comprend aucun des éléments suivants: (i) domaines α1, α2 et α3 de la chaîne α d'une molécule de CMH de classe I, (ii) microglobuline β2 d'une molécule de CMH de classe I, (iii) domaines α1 et α2 de la chaîne α d'une molécule de CMH de classe II, et (iv) domaines β1 et β2 de la chaîne β d'une molécule de CMH de classe II.

2. Molécule de fusion selon la revendication 1, dans laquelle la région transmembranaire est une région transmembranaire d'une molécule de CMH.

3. Molécule de fusion selon la revendication 1 ou 2, dans laquelle la région transmembranaire et la région cytoplasmique comprennent une séquence qui correspond à la région transmembranaire en liaison avec la région cytoplasmique d'une molécule de CMH.

4. Molécule de fusion selon l'une des revendications 1 à 3, dans laquelle la molécule de fusion comprend en outre une séquence de tête qui est apte à commander la translocation d'une protéine ou d'un peptide à travers une membrane.

5. Molécule de fusion selon la revendication 4, dans laquelle la séquence de tête est une séquence de tête d'une molécule de CMH.

6. Molécule de fusion selon la revendication 4 ou 5, dans laquelle la molécule de fusion présente la disposition suivante: séquence de tête à l'extrémité N-terminale / antigène / région transmembranaire /extrémité C-terminale de région cytoplasmique, tandis que les régions individuelles peuvent éventuellement être séparées les unes des autres par des séquences de liaison.

7. Acide nucléique, qui code pour une molécule de fusion selon l'une des revendications 1 à 6.

8. Cellule hôte, qui comprend un acide nucléique selon la revendication 7.

9. Composition pharmaceutique, qui comprend une ou plusieurs molécules de fusion selon l'une des revendications 1 à 6 et/ou un ou plusieurs acides nucléiques selon la revendication 7 et/ou une ou plusieurs cellules hôtes selon la revendication 8.

10. Composition pharmaceutique selon la revendication 9 sous forme d'un vaccin.

11. Procédé in vitro pour l'augmentation de la quantité de complexes de MHC/peptides dans une cellule, dans lequel le procédé comprend la fourniture d'une ou plusieurs molécules de fusion selon l'une des revendications 1 à 6 et/ ou d'un ou plusieurs acides nucléiques selon la revendication 7 pour la cellule.

12. Utilisation d'une ou plusieurs molécules de fusion selon l'une des revendications 1 à 6 et/ ou d'un ou plusieurs acides nucléiques selon la revendication 7 pour la préparation d'une composition pharmaceutique pour accroître la quantité de complexes de CMH/ peptides dans une cellule.

13. Procédé in vitro pour l'augmentation de la présentation de molécules de surface cellulaire sur des cellules qui sont capables de présenter des antigènes, en particulier des cellules B et des macrophages, tandis que le procédé comprend la fourniture d'une ou plusieurs molécules de fusion selon l'une des revendications 1 à 6 et/ou d'un ou plusieurs acides nucléiques selon la revendication 7 pour les cellules.

14. Utilisation d'une ou plusieurs molécules de fusion selon l'une des revendications 1 à 6 et/ ou d'un ou plusieurs acides nucléiques selon la revendication 7 pour la préparation d'une composition pharmaceutique pour augmenter la présentation de molécules de surface cellulaire sur des cellules qui sont capables de présenter des antigènes, en particulier des cellules B et des macrophages.

15. Procédé selon la revendication 11 ou 13 ou utilisation selon la revendication 12 ou 14, dans lequel(laquelle) l'augmentation de la quantité de complexes de CMH/peptides ou l'augmentation de la présentation de molécules de surface cellulaire renforce à son tour l'activation primaire des cellules T, en particulier des lymphocytes CD4⁺ et CD8⁺, qui réagissent vis-à-vis de l'antigène.

16. Utilisation d'une ou plusieurs molécules de fusion selon l'une des revendications 1 à 6 et/ou d'un ou plusieurs acides nucléiques selon la revendication 7 et/ou d'une ou plusieurs cellules hôtes selon la revendication 8 pour la préparation d'une composition pharmaceutique pour le déclenchement d'une réaction immune chez un être vivant.

17. Procédé in vitro pour la stimulation ou l'activation des cellules T, en particulier des lymphocytes CD4⁺ et CD8⁺, dans lequel le procédé comprend la fourniture aux cellules T d'une ou plusieurs molécules de fusion selon l'une des revendications 1 à 6 et/ou d'un ou plusieurs acides nucléiques selon la revendication 7 et/ou d'une ou plusieurs cellules hôtes selon la revendication 8.

18. Utilisation d'une ou plusieurs molécules de fusion selon l'une des revendications 1 à 6 et/ ou d'un ou plusieurs acides nucléiques selon la revendication 7 et/ou d'une ou plusieurs cellules hôtes selon la revendication 8 pour la préparation d'une composition pharmaceutique pour la stimulation ou l'activation des cellules T, en particulier des lymphocytes CD4⁺ et CD8⁺ chez un être vivant.

19. Utilisation d'une ou plusieurs molécules de fusion selon l'une des revendications 1 à 6 et/ ou d'un ou plusieurs acides nucléiques selon la revendication 7 et/ou d'une ou plusieurs cellules hôtes selon la revendication 8 pour la préparation d'une composition pharmaceutique pour le traitement, la vaccination ou l'immunisation d'un être vivant.
